# EUROPÄISCHE PATENTSCHRIFT

(11) **EP 2 240 431 B1**
(45) Veröffentlichungstag und Bekanntmachung des Hinweises auf die Patenterteilung: **25.01.2017**
(21) Anmeldenummer: 08857298.7
(22) Anmeldetag: 05.12.2008
(51) Int. Cl.: C07C 215/54, A61K 31/137, A61P 25/04

(54) **KRISTALLINE MODIFIKATIONEN VON (1R,2R)-3-(3-DIMETHYLAMINO-1-ETHYL-2-METHYL-PROPYL)-PHENOL**
CRYSTALLINE MODIFICATIONS OF (1R, 2R)-3-(3-DIMENTHYL AMINO-1-ETHYL-2-METHYL-PROPYL) PHENOL
MODIFICATIONS CRISTALLINES DE (1R,2R)-3-(3-DIMETHYLAMINO-1-ETHYL-2-METHYL-PROPYL)-PHÉNOL

(30) Priorität: 07.12.2007 EP 07023728
(43) Veröffentlichungstag der Anmeldung: 20.10.2010
(60) Teilanmeldung: 16203848.3
(73) Patentinhaber: Grünenthal GmbH, 52078 Aachen (DE)
(72) Erfinder: GRUSS, Michael, 52062 Aachen (DE)
(74) Vertreter: Brosch, Oliver
(86) Internationale Anmeldenummer: PCT/EP2008/010330
(87) Internationale Veröffentlichungsnummer: WO 2009/071310

(56) Entgegenhaltungen:
- EP-A- 0 693 475
- EP-A- 1 612 203

## Beschreibung

Die Erfindung betrifft kristalline Modifikationen von (1R,2R)-3-(3-Dimethylamino-1-ethyl-2-methyl-propyl)-phenol, pharmazeutische Zusammensetzungen, welche diese Modifikationen enthalten, sowie deren Verwendung.

(1R,2R)-3-(3-Dimethylamino-1-ethyl-2-methyl-propyl)-phenol ist ein synthetisches Analgetikum, das sich zur Behandlung von starken bis sehr starken, akuten und chronischen Schmerzen eignet. Die Verbindung kann dabei in Form ihrer freien Base oder in Form von pharmazeutisch verträglichen Salzen und Solvaten eingesetzt werden. Die Herstellung der Verbindung und ihrer Salze ist aus der EP-A-0 693 475 bekannt, wobei die Verbindung üblicherweise in Form ihres Salzes, beispielsweise in Form ihres Hydrochlorids erhalten wird.

Eine der vorliegenden Erfindung zugrunde liegende Aufgabe bestand darin, die Verbindung (1R,2R)-3-(3-Dimethylamino-1-ethyl-2-methyl-propyl)-phenol als solche, d.h. in Form der freien Base, mit guten Ausbeuten sowie guter Reinheit zugänglich zu machen.

Diese Aufgabe wurde durch den Gegenstand der Patentansprüche gelöst.

Es wurde überraschend gefunden, dass unter geeigneten Bedingungen die Verbindung (1R,2R)-3-(3-Dimethylamino-1-ethyl-2-methyl-propyl)-phenol in Form der nachstehend beschriebenen Polymorphe A, B und C erhalten werden kann.

Diese kristallinen Formen ermöglichen es, die Verbindung (1 R,2R)-3-(3-Dimethylamino-1-ethyl-2-methyl-propyl)-phenol in Form der freien Base, mit guten Ausbeuten sowie guter Reinheit zu erhalten. Diese Formen zeichnen sich ferner durch eine gute Handhabbarkeit aus und erleichtern eine exakte Dosierung des Wirkstoffs.

Darüber hinaus unterscheiden sich verschiedene Polymorphe desselben pharmazeutischen Wirkstoffs grundsätzlich in ihren Eigenschaften, woraus sich weitere Vorteile ergeben können.

Einerseits können die Vorteile auf eine spezielle physikalische Eigenschaft einer bestimmten Modifikation zurückgehen, beispielsweise bei ihrer Verarbeitung und Lagerung, wie z.B. thermodynamische Stabilität; Kristallmorphologie, insbesondere Gestalt, Größe, Farbe; Dichte; Schüttdichte; Härte; Deformierbarkeit;
kalorimetrisches Verhalten, insbesondere Schmelzpunkt; Löslichkeitseigenschaften; insbesondere intrinsische Löslichkeitsgeschwindigkeit und Gleichgewichtslöslichkeit; Hygroskopizität; relatives Feuchtigkeitsprofil; Klebrigkeit etc.

Andererseits kann die kristalline Modifikation auch verbesserte chemische Eigenschaften aufweisen. Beispielsweise ist bekannt, dass eine geringere Hygroskopizität zu einer verbesserten chemischen Stabilität und längeren Haltbarkeit von chemischen Verbindungen führen kann.

Ein Gegenstand der vorliegenden Erfindung betrifft eine kristalline Modifikation A von (1R,2R)-3-(3-Dimethylamino-1-ethyl-2-methyl-propyl)-phenol.

Diese erfindungsgemäße kristalline Modifikation A umfasst einen Röntgenbeugungsreflex bei 15,58±0,20 (2Θ), zusätzlich mindestens einen Röntgenbeugungsreflex ausgewählt aus der Gruppe bestehend aus 28,37±0,20 (2Θ) und 34,45±0,20 (2Θ) und zusätzlich mindestens einen Röntgenbeugungsreflex ausgewählt aus der Gruppe bestehend aus 13,71±0,20 (2Θ), 14,80±0,20 (2Θ), 16,89±0,20 (2Θ), 17,79±0,20 (2Θ), 18,45±0,20 (2Θ), 20,20±0,20 (2Θ), 20,92±0,20 (2Θ), 22,50±0,20 (2Θ), 24,37±0,20 (2Θ) und 25,33±0,20 (2Θ).

Weiterhin kann die erfindungsgemäße kristalline Modifikation A dadurch charakterisiert werden, dass sie neben dem Röntgenbeugungsreflex bei 15,58±0,20 (2Θ) und ggf. einem oder mehreren Röntgenbeugungsreflexen ausgewählt aus der Gruppe bestehend aus 28,37±0,20 (2Θ) und 34,45±0,20 (2Θ) und ggf. einem oder mehreren Röntgenbeugungsreflexen ausgewählt aus der Gruppe bestehend aus 13,71±0,20 (2Θ), 14,80±0,20 (2Θ), 16,89±0,20 (2Θ), 17,79±0,20 (2Θ), 18,45±0,20 (2Θ), 20,20±0,20 (2Θ), 20,92±0,20 (2Θ), 22,50±0,20 (2Θ), 24,37±0,20 (2Θ) und 25,33±0,20 (2Θ) zusätzlich mindestens einen Röntgenbeugungsreflex umfasst ausgewählt aus der Gruppe bestehend aus 14,11±0,20 (2Θ), 19,07±0,20 (2Θ), 21,12±0,20 (2Θ), 21,90±0,20 (2Θ), 22,21±0,20 (2Θ), 24,75±0,20 (2Θ), 27,32±0,20 (2Θ), 27,55±0,20 (2Θ), 29,90±0,20 (2Θ) und 30,68±0,20 (2Θ).

Die erfindungsgemäße kristalline Modifikation A kann ebenfalls dadurch charakterisiert werden, dass sie neben dem Röntgenbeugungsreflex bei 15,58±0,20 (2Θ) und ggf. einem oder mehreren Röntgenbeugungsreflexen ausgewählt aus der Gruppe bestehend aus 28,37±0,20 (2Θ) und 34,45±0,20 (2Θ) und ggf. einem oder mehreren Röntgenbeugungsreflexen ausgewählt aus der Gruppe bestehend aus 13,71±0,20 (2Θ), 14,80±0,20 (2Θ), 16,89±0,20 (2Θ), 17,79±0,20 (2Θ), 18,45±0,20 (2Θ), 20,20±0,20 (2Θ), 20,92±0,20 (2Θ), 22,50±0,20 (2Θ), 24,37±0,20 (2Θ) und 25,33±0,20 (2Θ) und ggf. einem oder mehreren Röntgenbeugungsreflexen ausgewählt aus der Gruppe bestehend aus 14,11±0,20 (2Θ), 19,07±0,20 (2Θ), 21,12±0,20 (2Θ), 21,90±0,20 (2Θ), 22,21±0,20 (2Θ), 24,75±0,20 (2Θ), 27,32±0,20 (2Θ), 27,55±0,20 (2Θ), 29,90±0,20 (2Θ) und 30,68±0,20 (2Θ) zusätzlich mindestens einen Röntgenbeugungsreflex umfasst ausgewählt aus der Gruppe bestehend aus 16,31±0,20 (2Θ), 23,30±0,20 (2Θ), 24,04±0,20 (2Θ), 28,05±0,20 (2Θ), 29,62 ±0,20 (2Θ), 30,28±0,20 (2Θ), 31,43±0,20 (2Θ), 32,21±0,20 (2Θ), 32,98±0,20 (2Θ), 33,41±0,20 (2Θ), 33,76±0,20 (2Θ), 34,17±0,20 (2Θ), 35,98±0,20 (2Θ), 36,24±0,20 (2Θ), 36,54±0,20 (2Θ), 36,87±0,20 (2Θ), 37,06±0,20 (2Θ), 37,48±0,20 (2Θ), 37,87±0,20 (2Θ), 38,64±0,20 (2Θ) und 39,48±0,20 (2Θ).

Bevorzugt lässt sich die erfindungsgemäße kristalline Modifikation A auch dadurch charakterisieren, dass sie einen oder mehrere der nachstehenden Röntgenbeugungsreflexe ausgewählt aus der Gruppe bestehend aus 10,93±0,20 (2Θ), 12,41±0,20 (2Θ) und 26,22±0,20 (2Θ) nicht umfasst.

Ebenfalls bevorzugt lässt sich die erfindungsgemäße kristalline Modifikation A ferner dadurch charakterisieren, dass sie einen oder mehreren der nachstehenden Röntgenbeugungsreflexe ausgewählt aus der Gruppe bestehend aus 8,10±0,20 (2Θ), 10,93±0,20 (2Θ), 11,83±0,20 (2Θ), 12,41±0,20 (2Θ), 26,22±0,20 (2Θ), 26,54±0,20 (2Θ) und 26,72±0,20 (2Θ) nicht umfasst.

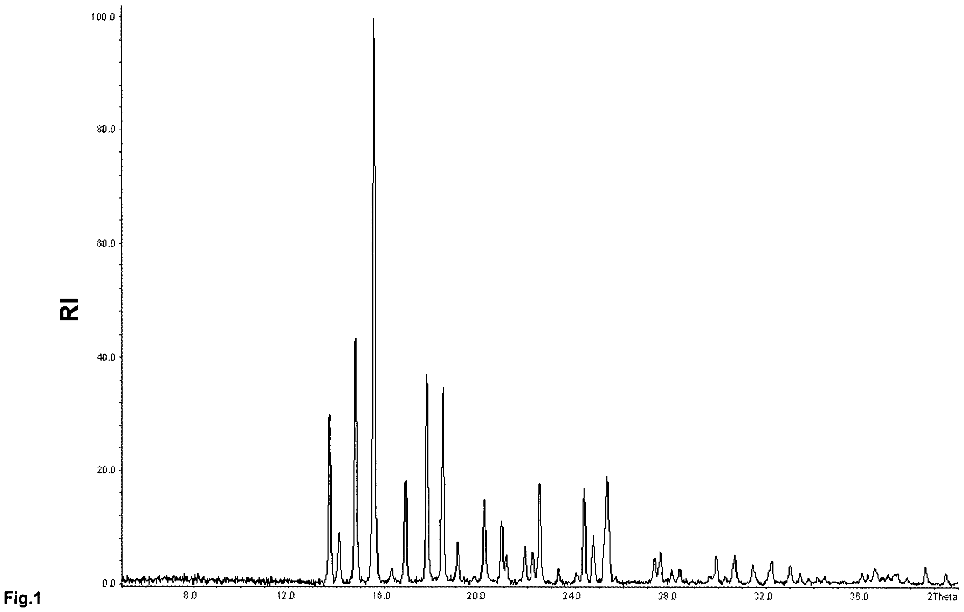

Figur 1 zeigt ein Röntgenpulverdiffraktogramm der Modifikation A.

Bevorzugt weist die erfindungsgemäße kristalline Modifikation A bei DSC Untersuchungen eine Endothermie mit Peaktemperatur bei 75-84°C, bevorzugter bei 76-83°C, noch bevorzugter bei 77-82°C und insbesondere bei 78-81 °C auf.

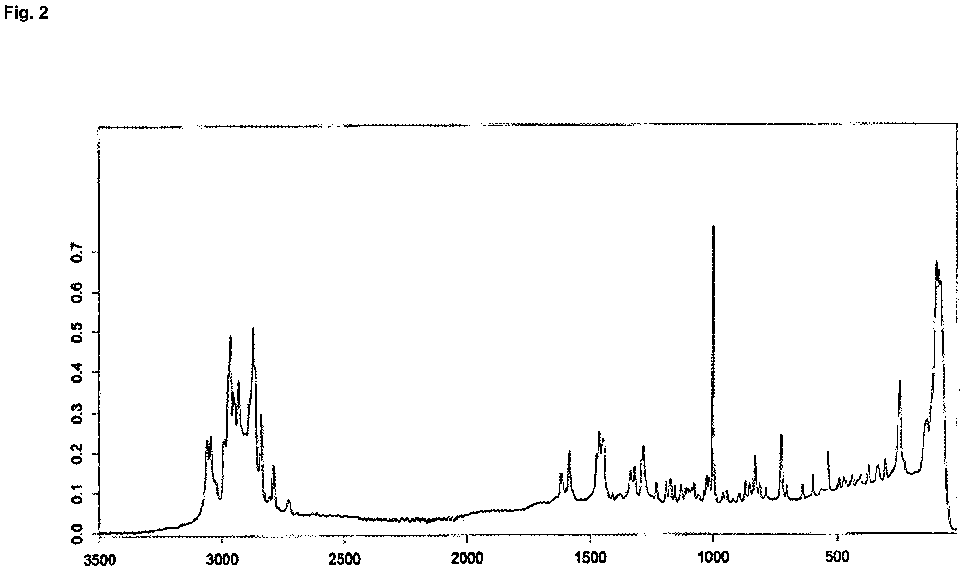

Die erfindungsgemäße kristalline Form A lässt sich ferner dadurch charakterisieren, dass sie eine oder mehre Raman-Banden umfasst ausgewählt aus der Gruppe bestehend aus 104±2 cm⁻¹, 249±2 cm⁻¹, 536±2 cm⁻¹, 724±2 cm⁻¹, 830±2 cm⁻¹, 999±2 cm⁻¹, 1283±2 cm⁻¹, 1462±2 cm⁻¹, 1584±2 cm⁻¹, 2790±2 cm⁻¹, 2839±2 cm⁻¹, 2873±2 cm⁻¹, 2933±2 cm⁻¹, 2965±2 cm⁻¹ und 3045±2 cm⁻¹. Figur 2 zeigt ein Raman-Spektrum der Modifikation A.

Ein weiterer Gegenstand der Erfindung betrifft ein Verfahren zur Herstellung der kristallinen Modifikation A umfassend die Schritte
(a) Einengen einer Lösung von (1R,2R)-3-(3-Dimethylamino-1-ethyl-2-methyl-propyl)-phenol, wobei das Lösemittel Methanol, Ethanol, 1-Propanol, 2-Propanol, Essigester, Aceton, Ethylmethylketon, Diethylether, tert-Butylmethylether, 1,4-Dioxan, Tetrahydrofuran, Acetonitril, Dichlormethan, Toluol, Dimethylformamid oder Dimethylsulfoxid ist, und
(b) Lagern des gemäß Schritt (a) erhaltenen Rückstands bei einer Temperatur von > 5 °C.
Zur Herstellung der kristallinen Modifikation A wird zunächst eine Lösung von (1 R,2R)-3-(3-Dimethylamino-1-ethyl-2-methyl-propyl)-phenol vorzugsweise vollständig eingeengt.

Das Einengen der Lösung kann nach üblichen, dem Fachmann bekannten Methoden erfolgen, beispielsweise am Rotationsverdampfer oder im Inertgasstrom, insbesondere im Argonstrom oder Stickstoffstrom.

Üblicherweise verbleibt nach dem Einengen ein vorzugsweise öliger Rückstand, der nach Lagerung bei einer Temperatur von > 5°C in Form der Modifikation A auskristallisiert. Im Allgemeinen reicht eine Lagerungsdauer von 24 Stunden aus.

Die weitere Aufarbeitung, sofern erforderlich, kann ebenfalls nach üblichen, dem Fachmann bekannten Methoden erfolgen, beispielsweise mittels Filtration, Waschen und/oder Trocknen.

Ein weiterer Gegenstand der Erfindung betrifft eine kristalline Modifikation A von (1R,2R)-3-(3-Dimethylamino-1-ethyl-2-methyl-propyl)-phenol, welche nach dem vorstehend beschriebenen Verfahren erhältlich ist.

Ein weiterer Gegenstand der vorliegenden Erfindung ist die kristalline Modifikation B von (1 R,2R)-3-(3-Dimethylamino-1-ethyl-2-methyl-propyl)-phenol.

Diese erfindungsgemäße kristalline Modifikation B von (1 R,2R)-3-(3-Dimethylamino-1-ethyl-2-methyl-propyl)-phenol umfasst einen Röntgenbeugungsreflex bei 29,06±0,20 (2Θ), zusätzlich mindestens einen Röntgenbeugungsreflex ausgewählt aus der Gruppe bestehend aus 19,50±0,20 (2Θ), 35,49±0,20 (2Θ) und 40,01±0,20 (2Θ) und zusätzlich mindestens einen Röntgenbeugungsreflex ausgewählt aus der Gruppe bestehend aus 14,11±0,20 (2Θ), 14,44±0,20 (2Θ), 16,08±0,20 (2Θ), 17,17±0,20 (2Θ), 17,43±0,20 (2Θ), 18,81 ±0,20 (2Θ), 20,24±0,20 (2Θ), 20,80±0,20 (2Θ), 22,00 ±0,20 (2Θ), 22,49±0,20 (2Θ), 23,40±0,20 (2Θ), 24,15±0,20 (2Θ), 24,51±0,20 (2Θ) und 29,89±0,20 (2Θ).

Weiterhin kann die erfindungsgemäße kristalline Modifikation B dadurch charakterisiert werden, dass sie neben dem Röntgenbeugungsreflex bei 29,06±0,20 (2Θ) und ggf. einem oder mehreren Röntgenbeugungsreflexen ausgewählt aus der Gruppe bestehend aus 19,50±0,20 (2Θ), 35,49±0,20 (2Θ) und 40,01±0,20 (2Θ) und ggf. einem oder mehreren Röntgenbeugungsreflexen ausgewählt aus der Gruppe bestehend aus 14,11±0,20 (2Θ), 14,44±0,20 (2Θ), 16,08±0,20 (2Θ), 17,17±0,20 (2Θ), 17,43±0,20 (2Θ), 18,81 ±0,20 (2Θ), 20,24±0,20 (2Θ), 20,80±0,20 (2Θ), 22,00 ±0,20 (2Θ), 22,49±0,20 (2Θ), 23,40±0,20 (2Θ), 24,15±0,20 (2Θ), 24,51±0,20 (2Θ) und 29,89±0,20 (2Θ) zusätzlich mindestens einen Röntgenbeugungsreflex umfasst ausgewählt aus der Gruppe bestehend aus 18,67±0,20 (2Θ), 25,24±0,20 (2Θ), 25,36±0,20 (2Θ), 27,58±0,20 (2Θ), 27,79±0,20 (2Θ), 30,11±0,20 (2Θ) und 31,00±0,20 (2Θ).

Die erfindungsgemäße kristalline Modifikation B kann auch dadurch charakterisiert werden, dass sie neben dem Röntgenbeugungsreflex bei 29,06±0,20 (2Θ) und ggf. einem oder mehreren Röntgenbeugungsreflexen ausgewählt aus der Gruppe bestehend aus 19,50±0,20 (2Θ), 35,49±0,20 (2Θ) und 40,01±0,20 (2Θ) und ggf. einem oder mehreren Röntgenbeugungsreflexen ausgewählt aus der Gruppe bestehend aus 14,11±0,20 (2Θ), 14,44±0,20 (2Θ), 16,08±0,20 (2Θ), 17,17±0,20 (2Θ), 17,43±0,20 (2Θ), 18,81 ±0,20 (2Θ), 20,24±0,20 (2Θ), 20,80±0,20 (2Θ), 22,00 ±0,20 (2Θ), 22,49±0,20 (2Θ), 23,40±0,20 (2Θ), 24,15±0,20 (2Θ), 24,51±0,20 (2Θ) und 29,89±0,20 (2Θ) und ggf. mindestens einen Röntgenbeugungsreflex umfasst ausgewählt aus der Gruppe bestehend aus 18,67±0,20 (2Θ), 25,24±0,20 (2Θ), 25,36±0,20 (2Θ), 27,58±0,20 (2Θ), 27,79±0,20 (2Θ), 30,11±0,20 (2Θ) und 31,00±0,20 (2Θ) mindestens einen Röntgenbeugungsreflex umfasst ausgewählt aus der Gruppe bestehend 22,85±0,20 (2Θ), 24,88±0,20 (2Θ), 30,31±0,20 (2Θ), 31,17±0,20 (2Θ), 31,54±0,20 (2Θ), 32,11±0,20 (2Θ), 32,45±0,20 (2Θ), 32,76±0,20 (2Θ), 33,61±0,20 (2Θ), 33,94±0,20 (2Θ), 35,95±0,20 (2Θ), 36,54±0,20 (2Θ), 37,12±0,20 (2Θ), 37,32±0,20 (2Θ), 37,75±0,20 (2Θ),38,13±0,20 (2Θ), 38,72±0,20 (2Θ) und 39,63±0,20 (2Θ).

Bevorzugt lässt sich die erfindungsgemäße kristalline Modifikation B auch dadurch charakterisieren, dass sie einen oder mehreren der nachstehenden Röntgenbeugungsreflexe ausgewählt aus der Gruppe bestehend aus 10,93±0,20 (2Θ), 12,41±0,20 (2Θ) und 26,22±0,20 (2Θ) nicht umfasst.

Ebenfalls bevorzugt lässt sich die erfindungsgemäße kristalline Modifikation B auch dadurch charakterisieren, dass sie einen oder mehreren der nachstehenden Röntgenbeugungsreflexe ausgewählt aus der Gruppe bestehend aus 8,10±0,20 (2Θ), 10,93±0,20 (2Θ), 11,83±0,20 (2Θ), 12,41±0,20 (2Θ), 26,22±0,20 (2Θ), 26,54±0,20 (2Θ) und 26,72±0,20 (2Θ) nicht umfasst.

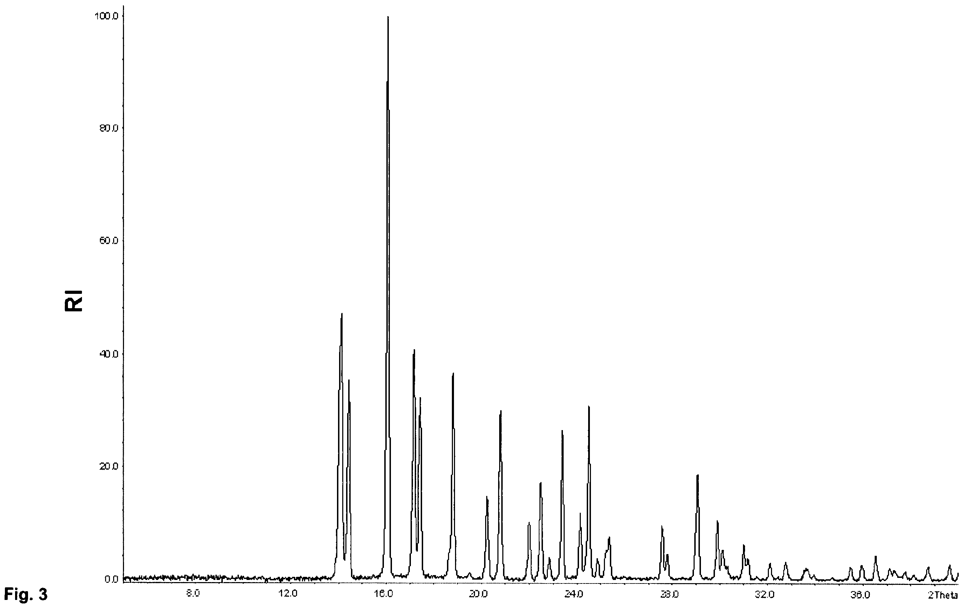

Figur 3 zeigt ein Röntgenpulverdiffraktogramm der Form B.

Bevorzugt weist die erfindungsgemäße kristalline Modifikation B bei DSC Untersuchungen eine Endothermie mit Peaktemperatur bei 87-93°C, bevorzugter bei 88-92°C, noch bevorzugter bei 89-91°C auf.

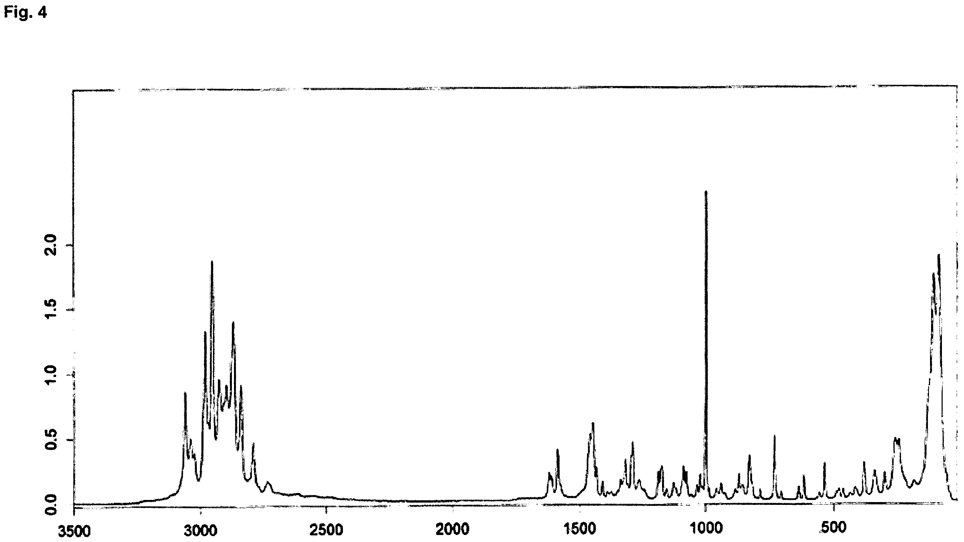

Die erfindungsgemäße kristalline Form B lässt sich ferner dadurch charakterisieren, dass sie eine oder mehre Raman-Banden umfasst ausgewählt aus der Gruppe bestehend aus 91±2 cm⁻¹, 112±2 cm⁻¹, 259±2 cm⁻¹, 381±2 cm⁻¹, 535±2 cm⁻¹, 730±2 cm⁻¹, 829±2 cm⁻¹, 999±2 cm⁻¹, 1088±2 cm⁻¹, 1173±2 cm⁻¹, 1288±2 cm⁻¹, 1445±2 cm⁻¹, 1585±2 cm⁻¹, 2790±2 cm⁻¹, 2838±2 cm⁻¹, 2869±2 cm⁻¹, 2925±2 cm⁻¹, 2952±2 cm⁻¹, 2980 cm⁻¹ und 3059±2 cm⁻¹. Figur 4 zeigt ein Raman-Spektrum der Form B.

Ein weiterer Gegenstand der Erfindung betrifft ein Verfahren zur Herstellung der kristallinen Modifikation B umfassend die Schritte
(a) Einengen einer Lösung von (1R,2R)-3-(3-Dimethylamino-1-ethyl-2-methyl-propyl)-phenol, wobei das Lösemittel Methanol, Ethanol, 1-Propanol, 2-Propanol, Essigester, Aceton, Ethylmethylketon, Diethylether, tert-Butylmethylether, 1,4-Dioxan, Tetrahydrofuran, Acetonitril, Dichlormethan, Toluol, Dimethylformamid oder Dimethylsulfoxid ist, und
(b1) Lagern des gemäß Schritt (a) erhaltenen Rückstands bei einer Temperatur von ≤ 5 °C, oder
(b2) Suspendieren des gemäß Schritt (a) erhaltenen Rückstands und Rühren dieser Suspension.
Zur Herstellung der kristallinen Modifikation B wird zunächst eine Lösung von (1 R,2R)-3-(3-Dimethylamino-1-ethyl-2-methyl-propyl)-phenol vorzugsweise vollständig eingeengt.

Das Einengen der Lösung kann nach üblichen, dem Fachmann bekannten Methoden erfolgen, beispielsweise am Rotationsverdampfer oder im Inertgasstrom, insbesondere im Argonstrom oder Stickstoffstrom.

Üblicherweise verbleibt nach dem Einengen ein vorzugsweise öliger Rückstand, der nach Lagerung bei einer Temperatur von ≤ 5°C in Form der Modifikation B auskristallisiert. Im Allgemeinen reicht eine Lagerungsdauer von 24 Stunden aus. Alternativ dazu, kann der vorzugsweise ölige Rückstand auch in einem geeigneten Suspensionsmedium aufgenommen und für einige Zeit gerührt werden. Als Suspensionsmedium eignen sich insbesondere Mischungen aus einem der vorstehend genannten Lösemittel mit Wasser oder einem gesättigten Kohlenwasserstoff, insbesondere n-Pentan, n-Hexan oder n-Heptan, wobei der Anteil des Lösemittels so zu wählen ist, dass der Rückstand nicht vollständig in Lösung geht.

Die Temperatur in Schritt (b) kann über einen breiten Bereich variieren, insbesondere im Bereich von 5-25°C, ebenso wie die Rührdauer, die von wenigen Minuten bis hin zu mehreren Wochen, insbesondere bis hin zu einer Woche reichen kann.

Ein weiterer Gegenstand der Erfindung betrifft ein Verfahren zur Herstellung der kristallinen Modifikation B umfassend den Schritt
(a) Präzipitieren von (1 R,2R)-3-(3-Dimethylamino-1-ethyl-2-methyl-propyl)-phenol aus Lösung, wobei das Lösemittel Methanol, Ethanol, 1-Propanol, 2-Propanol, Essigester, Aceton, Ethylmethylketon, Diethylether, tert-Butylmethylether, 1,4-Dioxan, Tetrahydrofuran, Acetonitril, Dichlormethan, Toluol, Dimethylformamid oder Dimethylsulfoxid ist.

Das Präzipitieren von (1R,2R)-3-(3-Dimethylamino-1-ethyl-2-methyl-propyl)-phenol aus der Lösung erfolgt dann mit Hilfe von Medien, in denen diese Verbindung nur schlecht löslich ist, wie beispielsweise gesättigten Kohlenwasserstoffen wie n-Pentan, n-Hexan und n-Heptan, und Wasser.

Die weitere Aufarbeitung, sofern erforderlich, kann ebenfalls nach üblichen, dem Fachmann bekannten Methoden erfolgen, beispielsweise mittels Filtration, Waschen und/oder Trocknen.

Die kristalline Modifikation B kann ferner auch durch Abkühlen einer Schmelze der kristallinen Modifikation A erhalten werden.

Ein weiterer Gegenstand der Erfindung betrifft eine kristalline Modifikation B von (1R,2R)-3-(3-Dimethylamino-1-ethyl-2-methyl-propyl)-phenol, welche nach den vorstehend beschriebenen Verfahren erhältlich ist.

Üblicherweise lässt sich Modifikation A durch schnellere Kristallisation und/oder bei höheren Temperaturen (wahrscheinlich über die amorphe Form als Zwischenstufe) erhalten. Modifikation B lässt sich üblicherweise durch langsamere Kristallisation und/oder bei niedrigeren Temperaturen (wahrscheinlich durch direkte Kristallisation) erhalten. Insbesondere im Temperaturbereich von 5-85°C, bevorzugt 5-50°C, stellt die Modifikation B die thermodynamisch stabilste Form dar.

Die thermodynamische Stabilität ist von Bedeutung. Durch Verwendung der stabilsten Modifikation in einem Medikament kann nämlich sichergestellt werden, dass während einer Lagerung keine polymorphe Umwandlung des Wirkstoffs in der pharmazeutischen Formulierung stattfindet. Dies ist von Vorteil, da sich anderenfalls infolge einer Umwandlung von einer weniger stabilen Modifikation in eine stabilere Modifikation die Eigenschaften des Medikaments ändern können. Im Hinblick auf die pharmakologischen Eigenschaften einer Darreichungsform könnte dies dazu führen, dass sich beispielsweise die Löslichkeit des Wirkstoffs ändert, womit eine Änderung des Freisetzungsverhaltens und somit auch eine Änderung der Bioverfügbarkeit einhergeht. Letztlich resultiert daraus eine unzureichende Lagerstabilität der Darreichungsform.

Ein weiterer Gegenstand der vorliegenden Erfindung ist auch die kristalline Modifikation C von (1R,2R)-3-(3-Dimethylamino-1-ethyl-2-methyl-propyl)-phenol.

Die kristalline Modifikation C von (1R,2R)-3-(3-Dimethylamino-1-ethyl-2-methylpropyl)-phenol umfasst mindestens einen Röntgenbeugungsreflex ausgewählt aus der Gruppe bestehend aus 10,93±0,20 (2Θ), 12,41±0,20 (2Θ) und 26,22±0,20 (2Θ), zusätzlich mindestens einen Röntgenbeugungsreflex ausgewählt aus der Gruppe bestehend aus 8,10±0,20 (2Θ), 11,83±0,20 (2Θ), 26,54±0,20 (2Θ) und 26,72±0,20 (2Θ) und zusätzlich mindestens einen Röntgenbeugungsreflex ausgewählt aus der Gruppe bestehend aus 13,71±0,20 (2Θ), 14,13±0,20 (2Θ), 14,82±0,20 (2Θ), 15,34±0,20 (2Θ), 15,59±0,20 (2Θ), 16,10±0,20 (2Θ),16,43±0,20 (2Θ), 16,91±0,20 (2Θ), 17,32±0,20 (2Θ),17,58±0,20 (2Θ), 17,82±0,20 (2Θ), 18,01±0,20 (2Θ), 18,46±0,20 (2Θ), 19,05±0,20 (2Θ), 20,23±0,20 (2Θ), 20,71±0,20 (2Θ), 20,94±0,20 (2Θ), 21,17±0,20 (2Θ), 21,90±0,20 (2Θ), 22,23±0,20 (2Θ), 22,52±0,20 (2Θ), 23,32±0,20 (2Θ), 24,12±0,20 (2Θ), 24,39±0,20 (2Θ), 24,92±0,20 (2Θ), 25,35±0,20 (2Θ), 27,33±0,20 (2Θ), 27,63±0,20 (2Θ), 27,84±0,20 (2Θ), 28,48±0,20 (2Θ), 29,64±0,20 (2Θ), 29,94±0,20 (2Θ), 30,54±0,20 (2Θ), 30,68±0,20 (2Θ), 31,03±0,20 (2Θ), 31,52±0,20 (2Θ), 32,29±0,20 (2Θ), 32,93±0,20 (2Θ), 33,66±0,20 (2Θ), 35,52±0,20 (2Θ), 36,05±0,20 (2Θ), 36,64±0,20 (2Θ), 37,54±0,20 (2Θ), 38,45±0,20 (2Θ), 39,15±0,20 (2Θ) und 40,05±0,20 (2Θ).

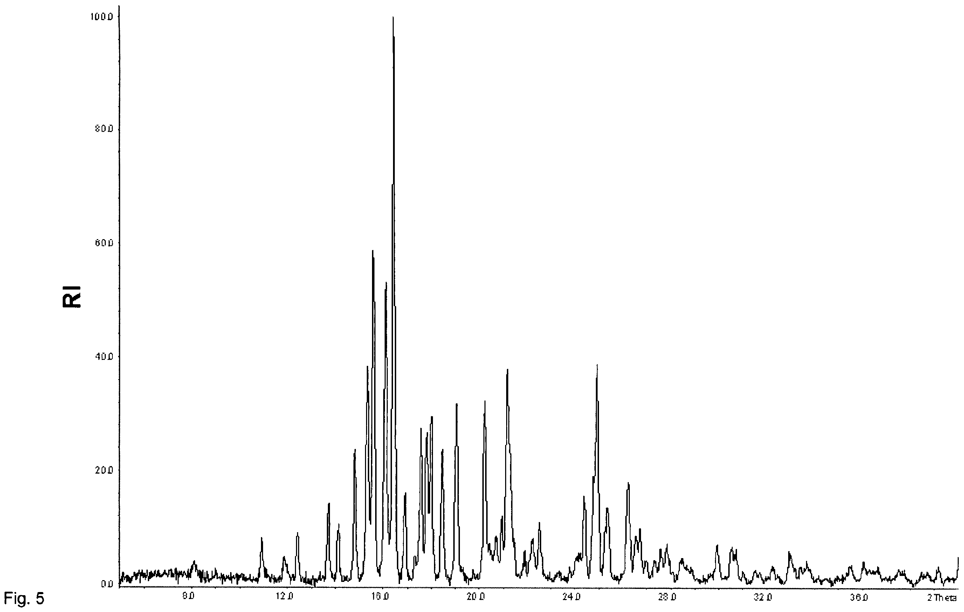

Figur 5 zeigt ein Röntgenpulverdiffraktogramm der Form C.

Bevorzugt weist die erfindungsgemäße kristalline Modifikation C bei DSC Untersuchungen eine Endothermie mit Peaktemperatur bei 75-84°C, bevorzugter bei 76-83°C, noch bevorzugter bei 77-82°C und insbesondere bei 78 bis 81 °C auf und/oder eine Endothermie mit Peaktemperatur bei 87-93°C, bevorzugter bei 88-92°C, noch bevorzugter bei 89-91 °C auf.

Ein weiterer Gegenstand der Erfindung betrifft ein Verfahren zur Herstellung der vorstehend beschriebenen Modifikation C umfassend die Schritte
(a) Schütteln einer Suspension enthaltend die kristalline Modifikation A und/oder die kristalline Modifikation B von (1R,2R)-3-(3-Dimethylamino-1-ethyl-2-methyl-propyl)-phenol, wobei das Suspensionsmedium Methanol oder Toluol ist, und
(b) Verdampfen des Suspensionsmediums im Luftstrom.

Vorzugsweise erfolgt das Schütteln der Suspension in Schritt a) bei einer Temperatur die oberhalb der Raumtemperatur (20-25°C) liegt, beispielsweise bei einer Temperatur im Bereich von > 25 bis 35 °C, vorzugsweise 30±3°C, besonders bevorzugt 30±2°C und insbesondere 30±1°C. Die Dauer des Schüttelvorgangs beträgt vorzugsweise 1-6 Stunden, bevorzugt 2-5 Stunden, noch weiter bevorzugt 3-4 Stunden.

Anschließend wird das Suspensionsmedium, ggf. nach Abkühlen der Suspension auf Raumtemperatur, im Luftstrom abgedampft und der so erhaltene Rückstand ggf. bei Raumtemperatur gelagert.

Die weitere Aufarbeitung, sofern erforderlich, kann ebenfalls nach üblichen, dem Fachmann bekannten Methoden erfolgen, beispielsweise mittels Filtration, Waschen und/oder Trocknen.

Ein weiterer Gegenstand der Erfindung betrifft eine kristalline Modifikation C von (1R,2R)-3-(3-Dimethylamino-1-ethyl-2-methyl-propyl)-phenol, welche nach dem vorstehend beschriebenen Verfahren erhältlich ist.

Ggf. können die erfindungsgemäßen Modifikationen A, B und C auch Cokristalle und Solvate bilden. Diese sind erfindungsgemäß jeweils umfasst.

Ebenfalls Gegenstand der vorliegenden Erfindung ist eine pharmazeutische Zusammensetzung enthaltend den Wirkstoff (1R,2R)-3-(3-Dimethylamino-1-ethyl-2-methyl-propyl)-phenol in kristalliner Form A, B oder C sowie wenigstens einen pharmazeutisch verträglichen Träger.

Bevorzugt kann die erfindungsgemäße pharmazeutische Zusammensetzung ein Polymorph ausgewählt aus der Gruppe bestehend aus Modifikation A, Modifikation B und Modifikation C enthalten.

Besonders bevorzugt kann die erfindungsgemäße pharmazeutische Zusammensetzung Modifikation A enthalten.

Ebenfalls besonders bevorzugt kann die erfindungsgemäße pharmazeutische Zusammensetzung Modifikation B enthalten.

Ein weiterer Gegenstand der vorliegenden Erfindung ist eine pharmazeutische Darreichungsform enthaltend eine erfindungsgemäße pharmazeutische Zusammensetzung wie vorstehend beschrieben.

Gegenstand der vorliegenden Erfindung ist auch eine erfindungsgemäße kristalline Modifikation von (1 R,2R)-3-(3-Dimethylamino-1-ethyl-2-methyl-propyl)-phenol, nämlich Modifikation A, B oder C wie vorstehend beschrieben als Arzneimittel. Ein weiterer Gegenstand der Erfindung betrifft die Verwendung wenigstens einer erfindungsgemäßen kristallinen Modifikation A, B oder C von (1 R,2R)-3-(3-Dimethylamino-1-ethyl-2-methyl-propyl)-phenol zur Herstellung eines Medikamentes zur Behandlung von Schmerz, insbesondere akutem Schmerz und chronischem Schmerz.

Neben wenigstens einer erfindungsgemäßen kristallinen Form A, Form B oder Form C oder einer Mischung aus wenigstens zwei dieser Formen kann das erfindungsgemäße Medikament üblicherweise weitere pharmazeutisch verträgliche Zusatz- oder Hilfsstoffe enthalten wie Trägermaterialien, Füllstoffe, Lösungsmittel, Verdünnungsmittel, Farbstoffe und/oder Bindemittel.

Die Auswahl der Hilfsstoffe sowie die einzusetzenden Mengen derselben hängt davon ab, ob das Arzneimittel oral, subkutan, parenteral, intravenös, intraperitoneal, intradermal, intramuskulär, intranasal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße kristalline Formen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen, kristallinen Formen verzögert freisetzen.

Die an den Patienten zu verabreichende Wirkstoffmenge kann variieren und ist beispielsweise abhängig vom Gewicht des Patienten, von der Applikationsart, der Indikation und dem Schweregrad der Erkrankung.
Figur 1 zeigt ein XRPD-Spektrum der kristallinen Modifikation A.
Figur 2 zeigt ein RAMAN-Spektrum der kristallinen Modifikation A.
Figur 3 zeigt ein XRPD-Spektrum der kristallinen Modifikation B.
Figur 4 zeigt ein RAMAN-Spektrum der kristallinen Modifikation B.
Figur 5 zeigt ein XRPD-Spektrum der kristallinen Modifikation C.

Im Folgenden wird die Erfindung anhand von Beispielen erläutert. Diese Erläuterungen sind lediglich beispielhaft und schränken den allgemeinen Erfindungsgedanken nicht ein.

### Beispiele:

### Abkürzungen

- RT: Raumtemperatur, vorzugsweise 20-25°C
- TBME: tert.-Butylmethylether
- EtOH: Ethanol
- MEK: 2-Butanon
- THF: Tetrahydofuran
- 2PrOH: 2-Propanol
- EtOAc: Ethylacetat
- MeCN: Acetonitril
- DMSO: Dimethylsulfoxid
- DMF: Dimethylformamid
- IR: Infrarot
- Min: Minute
- Sec: Sekunde

Sofern nicht anders angegeben beziehen sich Lösemittelgemische immer auf Volumen/Volumen.

### A) Synthese von Modifikation A

### A1)

16,689 g (1R,2R)-3-(3-Dimethylamino-1-ethyl-2-methyl-propyl)-phenol, Hydrochlorid (erhältlich analog zur Vorschrift gemäß EP A 0 693 475) wurden in einem 250 ml Dreihalskolben in 81 ml destilliertem Wasser gelöst und bei Raumtemperatur mit 32 Gew.-% iger Natronlauge versetzt bis ein pH-Wert von 11 erreicht war (ca. 7 ml). Bereits nach Zugabe weniger ml fiel eine weiße, zähe Substanz aus, die in ca. 16 ml Essigester gelöst wurde. Nach beendeter Zugabe wurde eine weiße Suspension erhalten, die für 1 Stunde nachgerührt wurde. Der pH-Wert fiel dabei auf 10 und es wurden weitere 0,5 ml Natronlauge zugegeben. Anschließend wurde die ausgefällte Base mit insgesamt 288 ml Essigester extrahiert. Die vereinigten organischen Phasen wurden dann mit ca. 32 ml Wasser gewaschen, über Magnesiumsulfat getrocknet und im Vakuum am Rotationsverdampfer bis zur Trockne eingeengt.

Im Kolben blieb ein gelbes Öl zurück, das auch bei Raumtemperatur nicht kristallisierte. Die Kristallisation wurde daraufhin durch Ankratzen des Kolbens mit einem Spatel initiiert und das Öl kristallisierte innerhalb von wenigen Minuten in Form eines gelben Rückstands aus. Dieser Rückstand wurde im Mörser zerkleinert und es wurde ein fast weißer kristalliner Feststoff der Modifikation A erhalten, der durch ¹H-NMR, DSC, TG-FTIR, XRPD, Raman und HPLC charakterisiert wurde.

Ein Teil des so erhaltenen kristallinen Feststoffs wurde wie folgt umkristallisiert:
30 mg der Modifikation A wurden in ein 20 ml Gefäß eingewogen, mit 6 ml 2-Propanol versetzt und bei 30 °C und 400 Umdrehungen pro Minute für 4 Stunden geschüttelt. Anschließend wird das Lösemittel im Luftstrom bei 23°C abgedampft. Es wurde ein weißer kristalliner Feststoff der Form A erhalten.

### A2)

200 mg ① gemäß B) wurden in 25 mL Acetonitril gelöst. Anschließend wurde das Lösemittel im Vakuum am Rotationsverdampfer entfernt. Es blieb ein farbloses Öl zurück. Zu diesem Öl wurden ca. 1 mg Impfkristalle der Form A gegeben und die Probe bei Raumtemperatur für 2 Tage gelagert. Es wurde ein kristalliner Feststoff der Form A erhalten.

### B) Synthese von Modifikation B

### B1)

Aus 3300 g (2R,3R)-[3-(3-Methoxy-phenyl)-2-methyl-pentyl]-dimethylamin, Hydrobromid (erhältlich analog zur Vorschrift gemäß EP A 0 693 475) in Methylcyclohexan wurden zunächst mit 45 Gew.-%iger Natronlauge (Säureverbrauchszahl = 4,11 mol/Kg) 3192 g (2R,3R)-[3-(3-Methoxy-phenyl)-2-methyl-pentyl]-dimethylamin in Form der freien Base erhalten.

In Methylcyclohexan wurden 18,9 kg Methansulfonsäure und 2458 g D,L Methionin vorgelegt und dann 3192 g (2R,3R)-[3-(3-Methoxy-phenyl)-2-methyl-pentyl]-dimethylamin zugegeben und die Mischung bei 82°C für 18 Stunden gerührt. Anschließend wurde mit 10,3 L Wasser bei höchstens 80°C verdünnt und 9 L Methylcyclohexan zugegeben. Bei höchstens 42°C wurden 17,3 L Ammoniak dazugegeben bis der pH 8.8 betrug. Bei 45 °C erfolgte eine Phasentrennung und die organische Phase wurde bei 40°C mit 3,2 g (1R,2R)-3-(3-Dimethylamino-1-ethyl-2-methyl-propyl)-phenol versetzt und 1 Stunde bei 36°C gerührt. Anschließend wurde langsam auf 5°C abgekühlt und eine Stunde weiter gerührt, der gebildete Niederschlag abgesaugt, mit 12 L Methylcyclohexan gewaschen und im Trockenschrank getrocknet. Es wurden 2685 g (89,5 %) (1 R,2R)-3-(3-Dimethylamino-1-ethyl-2-methyl-propyl)-phenol in der Modifikation B erhalten.

Die so erhaltene Verbindung der Modifikation B wird in den nachfolgenden Versuchen mit ① bezeichnet.

### C) Synthese von Modifikation C

### C1)

48,6 mg der Modifikation B wurden in 10 mL Methanol suspendiert und bei 30 °C und 400 Umdrehungen pro Minute für 4 Stunden mit einem Vortexer geschüttelt. Nach dem Abkühlen auf RT wurde das Lösemittel bei RT im Luftstrom abgedampft.

Nach 24 Stunden lag der Rückstand als Gemisch von Öl und Feststoff vor. Nach weiterer Lagerung (72 Stunden, verschlossen, Raumtemperatur) wurde ein weißer Feststoff erhalten.

### C2)

30,23 mg der Modifikation A wurden in 6 mL Toluol suspendiert und bei 30 °C und 400 Umdrehungen pro Minute für 4 Stunden mit einem Vortexer geschüttelt. Nach dem Abkühlen auf RT wurde das Lösemittel bei 23°C im Luftstrom abgedampft. Es wurde ein weißer Feststoff erhalten.

Die bei DSC Untersuchungen gefundenen Peaktemperaturen für die gemäß C1) und C2) erhaltenen Produkte lagen im Bereich von 78-82 °C bzw. 87-90 °C und somit im Bereich der für Modifikationen A und B gefundenen Peaktemperaturen. Bei den Produkten könnte es sich daher um eine Mischung der Formen A und B handeln. Das Pulverdiffraktogramm zeigt jedoch Röntgenbeugungsreflexe, die nicht von einer Mischung der Modifikationen A und B stammen können.

### Kristallisationsversuche:

### Beispiel 1:

Amorphes (1 R,2R)-3-(3-Dimethylamino-1-ethyl-2-methyl-propyl)-phenol wurde durch schnelle Verdampfung einer Lösung der Verbindung am Rotationsverdampfer erhalten. Die öligen Rückstände wurden bei RT oder bei 5 °C gelagert. Innerhalb von 24 Stunden kristallisierten alle Proben. Bei RT wurde Modifikation A oder Mischungen aus Modifikation A und Modifikation B enthalten. Bei tieferen Temperaturen (5°C) wurde Modifikation B erhalten.
1.1) 109,1 mg ① wurden in 2 ml TBME gelöst. Das Lösemittel wurde am Rotationsverdampfer entfernt. Es wurde ein farbloses Öl erhalten. Der Rückstand wurde über Nacht bei RT gelagert. Es wurde eine Mischung aus Modifikation A und B erhalten.
1.2) 100 mg ① wurden in 2 ml EtOH gelöst. Das Lösemittel wurde am Rotationsverdampfer entfernt. Es wurde ein farbloses Öl erhalten. Der Rückstand wurde über Nacht bei RT gelagert. Es wurde Modifikation A erhalten.
1.3) 105,6 mg ① wurden in 2 ml EtOAc gelöst. Das Lösemittel wurde am Rotationsverdampfer entfernt. Es wurde ein farbloses Öl erhalten. Der Rückstand wurde über Nacht bei RT gelagert. Es wurde eine Mischung aus Modifikation A und B erhalten.
1.4) 100,9 mg ① wurden in 2 ml Aceton gelöst. Das Lösemittel wurde am Rotationsverdampfer entfernt. Es wurde ein farbloses Öl erhalten. Der Rückstand wurde über Nacht bei 5°C gelagert. Es wurde Modifikation B erhalten.
1.5) 100,0 mg ① wurden in 2 ml MEK gelöst. Das Lösemittel wurde am Rotationsverdampfer entfernt. Es wurde ein farbloses Öl erhalten. Der Rückstand wurde über Nacht bei 5°C gelagert. Es wurde Modifikation B erhalten.
1.6) 99,5 mg ① wurden in 2 ml THF gelöst. Das Lösemittel wurde am Rotationsverdampfer entfernt. Es wurde ein farbloses Öl erhalten. Der Rückstand wurde über Nacht bei 5°C gelagert. Es wurde Modifikation B erhalten.

### Beispiel 2:

Amorphes (1R,2R)-3-(3-Dimethylamino-1-ethyl-2-methyl-propyl)-phenol wurde durch schnelle Verdampfung einer Lösung der Verbindung am Rotationsverdampfer oder im Stickstoffstrom erhalten. Die öligen Rückstände wurden in verschiedenen Lösemitteln suspendiert und die so erhaltenen Mischungen bei RT oder bei 5 °C gerührt. Es wurde keine Bildung von Solvaten in den ausgewählten Lösemitteln beobachtet.
2.1) 96,9 mg ① wurden in 1 ml THF gelöst. Die Lösung wurde filtriert und nachfolgend das Lösemittel bei RT unter starkem Stickstoffstrom entfernt. 500 µl TBME wurden zu dem so erhaltenen Rückstand gegeben. Die Mischung wurde bei RT für die Dauer von 2 Wochen gerührt. Alle festen Bestandteile waren gelöst.
2.2) 104,2 mg ① wurden in 1 ml THF gelöst. Die Lösung wurde filtriert und nachfolgend das Lösemittel bei RT unter starkem Stickstoffstrom entfernt. 500 µl TBME wurden zu dem so erhaltenen Rückstand gegeben. Die Mischung wurde bei RT für die Dauer von 2 Wochen gerührt. Alle festen Bestandteile waren gelöst.
2.3) 99,9 mg ① wurden in 1 ml THF gelöst. Die Lösung wurde filtriert und nachfolgend das Lösemittel bei RT unter starkem Stickstoffstrom entfernt. 500 µl H₂O wurden zu dem so erhaltenen Rückstand gegeben. Die Mischung wurde bei RT für die Dauer von 1 Woche gerührt. Der resultierende kristalline Rückstand wurde abfiltriert. Es wurde Modifikation B erhalten.
2.4) 95,3 mg ① wurden in 1 ml THF gelöst. Die Lösung wurde filtriert und nachfolgend das Lösemittel bei RT unter starkem Stickstoffstrom entfernt. 500 µl IPE wurden zu dem so erhaltenen Rückstand gegeben. Die Mischung wurde bei RT für die Dauer von 2 Wochen gerührt. Alle festen Bestandteile waren gelöst.
2.5) 101,7 mg ① wurden in 1 ml THF gelöst. Die Lösung wurde filtriert und nachfolgend das Lösemittel bei RT unter starkem Stickstoffstrom entfernt. 500 µl H₂O/EtO (1:1) wurden zu dem so erhaltenen Rückstand gegeben. Die Mischung wurde bei RT für die Dauer von 1 Woche gerührt. Der resultierende kristalline Rückstand wurde abfiltriert. Es wurde Modifikation B erhalten.
2.6) 101,0 mg ① wurden in 1 ml THF gelöst. Die Lösung wurde filtriert und nachfolgend das Lösemittel bei RT unter starkem Stickstoffstrom entfernt. 500 µl Aceton/EtOH (1:1) wurden zu dem so erhaltenen Rückstand gegeben. Die Mischung wurde bei RT für die Dauer von 2 Wochen gerührt. Es bildeten sich zwei flüssige Phasen.
2.7) 96,9 mg ① wurden in 1 ml THF gelöst. Die Lösung wurde filtriert und nachfolgend das Lösemittel bei RT unter starkem Stickstoffstrom entfernt. 500 µl TBME wurden zu dem so erhaltenen Rückstand gegeben. Die Mischung wurde bei 5°C für die Dauer von 2 Wochen gerührt. Alle festen Bestandteile waren gelöst.
2.8) 109,0 mg ① wurden in 1 ml THF gelöst. Die Lösung wurde filtriert und nachfolgend das Lösemittel bei RT unter starkem Stickstoffstrom entfernt. 500 µl Heptan/TBME (1:1) wurden zu dem so erhaltenen Rückstand gegeben. Die Mischung wurde bei einer Temperatur von 5°C für die Dauer von 1 Woche gerührt. Der resultierende kristalline Rückstand wurde abfiltriert. Es wurde Modifikation B erhalten.
2.9) 98,5 mg ① wurden in 1 ml THF gelöst. Die Lösung wurde filtriert und nachfolgend das Lösemittel bei RT unter starkem Stickstoffstrom entfernt. 500 µl H₂O wurden zu dem so erhaltenen Rückstand gegeben. Die Mischung wurde bei einer Temperatur von 5°C für die Dauer von 1 Woche gerührt. Der resultierende kristalline Rückstand wurde abfiltriert. Es wurde eine Mischung aus den Modifikationen A und B erhalten.
2.10) 100,7 mg ① wurden in 1 ml THF gelöst. Die Lösung wurde filtriert und nachfolgend das Lösemittel bei RT unter starkem Stickstoffstrom entfernt. 500 µl IPE wurden zu dem so erhaltenen Rückstand gegeben. Die Mischung wurde bei 5°C für die Dauer von 2 Wochen gerührt. Alle festen Bestandteile waren gelöst.
2.11) 96,7 mg ① wurden in 1 ml THF gelöst. Die Lösung wurde filtriert und nachfolgend das Lösemittel bei RT unter starkem Stickstoffstrom entfernt. 500 µl EtOH/H₂O (1:1) wurden zu dem so erhaltenen Rückstand gegeben. Die Mischung wurde bei einer Temperatur von 5°C für die Dauer von 1 Woche gerührt. Der resultierende kristalline Rückstand wurde abfiltriert. Es wurde Modifikation B erhalten.
2.12) 105,1 mg ① wurden in 1 ml THF gelöst. Die Lösung wurde filtriert und nachfolgend das Lösemittel bei RT unter starkem Stickstoffstrom entfernt. 500 µl Aceton/H₂O (1:1) wurden zu dem so erhaltenen Rückstand gegeben beigegeben. Die Mischung wurde bei einer Temperatur von 5°C für die Dauer von 1 Woche gerührt. Der resultierende kristalline Rückstand wurde abfiltriert. Es wurde Modifikation B erhalten.

### Beispiel 3:

Es wurden Kristallisationsversuche mittels Dampfdiffusion durchgeführt, in denen gesättigte Kohlenwasserstoffe und Ether als Fällungsmittel verwendet wurden. Hierbei wurde nur in einem Fall ein kristalliner Niederschlag, nämlich die Modifikation B, erhalten.
3.1) 200 mg ① wurden in 2 ml 2PrOH gelöst. Die Lösung wurde in einer gesättigten n-Hexan Atmosphäre bei RT für die Dauer von 8 Wochen gelagert. Es wurde kein Niederschlag erhalten.
3.2) 200 mg ① wurden in 2 ml EtOAc gelöst. Die Lösung wurde in einer gesättigten n-Hexan Atmosphäre bei RT für die Dauer von 8 Wochen gelagert. Es wurde kein Niederschlag erhalten.
3.3) 200 mg ① wurden in 2 ml Toluol gelöst. Die Lösung wurde in einer gesättigten n-Hexan Atmosphäre bei RT für die Dauer von 8 Wochen gelagert. Es wurde kein Niederschlag erhalten.
3.4) 200 mg ① wurden in 2 ml THF gelöst. Die Lösung wurde in einer gesättigten n-Hexan Atmosphäre bei RT für die Dauer von 8 Wochen gelagert. Der gebildete kristalline Niederschlag wurde abfiltriert. Es wurde Modifikation B erhalten.
3.5) 200 mg ① wurden in 2 ml 2PrOH gelöst. Die Lösung wurde in einer gesättigten IPE Atmosphäre bei RT für die Dauer von 8 Wochen gelagert. Es wurde kein Niederschlag erhalten.
3.6) 200 mg ① wurden in 2 ml EtOAc gelöst. Die Lösung wurde in einer gesättigten IPE Atmosphäre bei RT für die Dauer von 8 Wochen gelagert. Es wurde kein Niederschlag erhalten.
3.7) 200 mg ① wurden in 2 ml Toluol gelöst. Die Lösung wurde in einer gesättigten IPE Atmosphäre bei RT für die Dauer von 8 Wochen gelagert. Es wurde kein Niederschlag erhalten.
3.8) 200 mg ① wurden in 2 ml THF gelöst. Die Lösung wurde in einer gesättigten IPE Atmosphäre bei RT für die Dauer von 8 Wochen gelagert. Es wurde kein Niederschlag erhalten.
3.9) 200 mg ① wurden in 2 ml 2-PrOH gelöst. Die Lösung wurde in einer gesättigten TBME Atmosphäre bei RT für die Dauer von 8 Wochen gelagert. Es wurde kein Niederschlag erhalten.
3.10) 200 mg ① wurden in 2 ml EtOAc gelöst. Die Lösung wurde in einer gesättigten TBME Atmosphäre bei RT für die Dauer von 8 Wochen gelagert. Es wurde kein Niederschlag erhalten.
3.11) 200 mg ① wurden in 2 ml Toluol gelöst. Die Lösung wurde in einer gesättigten TBME Atmosphäre bei RT für die Dauer von 8 Wochen gelagert. Es wurde kein Niederschlag erhalten.
3.12) 200 mg ① wurden in 2 ml THF gelöst. Die Lösung wurde in einer gesättigten TBME Atmosphäre bei RT für die Dauer von 8 Wochen gelagert. Es wurde kein Niederschlag erhalten.
3.13) 200 mg ① wurden in 1 ml EtOAc gelöst. Die Lösung wurde in einer gesättigten Cyclohexan Atmosphäre bei RT für die Dauer von 1 Woche gelagert. Es wurde kein Niederschlag erhalten. Die Probe wurde bei 5°C für die Dauer von zwei Wochen gelagert. Es wurde kein Niederschlag erhalten.
3.14) 200 mg ① wurden in 3 ml MeCN gelöst. Die Lösung wurde in einer gesättigten Cyclohexan Atmosphäre bei RT für die Dauer von 1 Woche gelagert. Es wurde kein Niederschlag erhalten. Die Probe wurde bei 5°C für die Dauer von zwei Wochen gelagert. Es wurde kein Niederschlag erhalten.
3.15) 200 mg ① wurden in 1 ml DMSO gelöst. Die Lösung wurde in einer gesättigten Cyclohexan Atmosphäre bei RT für die Dauer von 3 Wochen gelagert. Es wurde kein Niederschlag erhalten.
3.16) 200 mg ① wurden in 1 ml EtOAc gelöst. Die Lösung wurde in einer gesättigten Pentan Atmosphäre bei RT für die Dauer von 1 Woche gelagert. Es wurde kein Niederschlag erhalten. Die Probe wurde bei 5°C für die Dauer von zwei Wochen gelagert. Es wurde kein Niederschlag erhalten.
3.17) 200 mg ① wurden in 3 ml MeCN gelöst. Die Lösung wurde in einer gesättigten Pentan Atmosphäre bei RT für die Dauer von 1 Woche gelagert. Es wurde kein Niederschlag erhalten. Die Probe wurde bei 5°C für die Dauer von zwei Wochen gelagert. Es wurde kein Niederschlag erhalten.
3.18) 200 mg ① wurden in 1 ml DMSO gelöst. Die Lösung wurde in einer gesättigten Pentan Atmosphäre bei RT für die Dauer von 3 Wochen gelagert. Es wurde kein Niederschlag erhalten.

### Beispiel 4:

4.1) 100 mg ① wurden in 1 ml EtOAc gelöst. Schrittweise wurden 2 ml Aceton zugegeben bis die Lösung trübe wurde. Die Probe wurde bei 5 °C für die Dauer von 10 Tagen gelagert. Es wurde keine Veränderung beobachtet.
4.2) 100 mg ① wurden in 0,5 ml 1,4-Dioxan gelöst. Schrittweise wurden 2 ml H₂O zugegeben bis die Lösung trüb wurde und ein klebriges Harz ausfiel. Die Probe wurde bei 5°C über Nacht gelagert. Nach Kratzen mit einem Spatel kristallisierte das Harz aus und der kristalline Feststoff wurde abfiltriert. Es wurde Modifikation B erhalten.
4.3) 100 mg ① wurden in 0,5 ml EtOAc gelöst. Schrittweise wurden 6 ml Heptan zugegeben bis die Lösung trüb wurde und ein farbloser Feststoff ausfiel. Die Probe wurde bei 5°C für die Dauer von 6 Tagen gelagert und der erhaltene Feststoff wurde abfiltriert. Es wurde ein kristallines Pulver erhalten.
4.4) 100 mg ① wurden in 1 ml Dioxan gelöst. Schrittweise wurden 3 ml Heptan zugegeben bis die Lösung trübe wurde. Die Probe wurde bei 5 °C für die Dauer von 1 Woche gelagert. Es wurde keine Veränderung beobachtet.
4.5) 100 mg ① wurden in 1 ml Dioxan gelöst. Schrittweise wurden 11 ml iBuOAc zugegeben. Es wurde kein Niederschlag erhalten. Die Probe wurde bei 5 °C für die Dauer von 1 Woche gelagert. Es wurde keine Veränderung beobachtet.
4.6) 100 mg ① wurden in 1 ml EtOAc gelöst. Schrittweise wurden 1 ml Pentan zugegeben bis die Lösung trübe wurde. Die Probe wurde bei 5 °C für die Dauer von 1 Woche gelagert. Es wurde keine Veränderung beobachtet.
4.7) 100 mg ① wurden in 2,5 ml MeOH gelöst. Schrittweise wurden 3 ml H₂O zugegeben bis die Lösung trüb wurde und ein farbloser Feststoff ausfiel. Die Probe wurde bei RT für die Dauer von 1 Woche gelagert und der erhaltene Feststoff wurde abfiltriert. Es wurde ein kristallines Pulver der Modifikation A erhalten.
4.8) 100 mg ① wurden in 500 µl 2PrOH gelöst. Schrittweise wurden 3 ml H₂O zugegeben und die Mischung bei RT für die Dauer von 5 Tagen gerührt. Der erhaltene Feststoff wurde abfiltriert. Es wurde ein kristallines Pulver der Modifikation B erhalten.
4.9) 100 mg ① wurden in 500 µl EtOH gelöst. Schrittweise wurden 3 ml H₂O zugegeben und die Mischung bei RT für die Dauer von 5 Tagen gerührt. Der erhaltene Feststoff wurde abfiltriert. Es wurde ein kristallines Pulver der Modifikation B erhalten.
4.10) 100 mg ① wurden in 1 ml DMF gelöst. Schrittweise wurden 2 ml H₂O zugegeben und die Mischung bei RT für die Dauer von 5 Tagen gerührt. Der erhaltene Feststoff wurde abfiltriert. Es wurde ein kristallines Pulver der Modifikation B erhalten.
4.11) 100 mg ① wurden in 1 ml DMSO gelöst. Schrittweise wurden 1 ml H₂O zugegeben und die Mischung bei RT für die Dauer von 5 Tagen gerührt. Der erhaltene Feststoff wurde abfiltriert. Es wurde ein kristallines Pulver der Modifikation B erhalten.
4.12) 100 mg ① wurden in 500 µl EtOAc gelöst. Schrittweise wurden 2 ml Pentan zugegeben und die Mischung bei RT für die Dauer von einigen Stunden gerührt. Es bildete sich ein klebriger Feststoff. Die Probe wurde bei 5°C für die Dauer von 3 Wochen gelagert und der erhaltene Feststoff wurde abfiltriert.
4.13) 100 mg ① wurden in 500 µl EtOAc gelöst. Schrittweise wurden 2 ml n-Hexan zugegeben und die Mischung bei RT für die Dauer von einigen Stunden gerührt. Es bildete sich ein klebriger Feststoff. Die Probe wurde bei 5°C für die Dauer von 2 Wochen gelagert und der erhaltene Feststoff wurde abfiltriert. Es wurde ein kristallines Pulver der Modifikation B erhalten.
4.14) 100 mg ① wurden in 500 µl EtOAc gelöst. Schrittweise wurden 2 ml n-Heptan zugegeben und die Mischung bei RT für die Dauer von einigen Stunden gerührt. Es bildete sich ein klebriger Feststoff. Die Probe wurde bei 5°C für die Dauer von 2 Wochen gelagert und der erhaltene Feststoff wurde abfiltriert. Es wurde ein kristallines Pulver der Modifikation B erhalten.
4.15) 100 mg ① wurden in 500 µl EtOAc gelöst. Schrittweise wurden 2 ml Cyclohexan zugegeben und die Mischung bei RT für die Dauer von einigen Stunden gerührt. Es bildete sich ein klebriger Feststoff. Die Probe wurde bei 5°C für die Dauer von 2 Wochen gelagert und der erhaltene Feststoff wurde abfiltriert. Es wurde ein kristallines Pulver erhalten.

### Beispiel 5:

5.1) Die nach Beispiel 2.1) erhaltene Lösung wurde bei RT in einem offenen Gefäß gelagert, um das Lösemittel abzudampfen. Nach 1 Woche wurde ein kristalliner Feststoff der Modifikation A erhalten.
5.2) Die nach Beispiel 2.2) erhaltene Lösung wurde bei RT in einem offenen Gefäß gelagert, um das Lösemittel abzudampfen. Nach 1 Woche wurde ein kristalliner Feststoff der Modifikation A erhalten.
5.3) Die nach Beispiel 2.4) erhaltene Lösung wurde bei RT in einem offenen Gefäß gelagert, um das Lösemittel abzudampfen. Nach 1 Woche wurde ein kristalliner Feststoff der Modifikation A erhalten.
5.4) Die nach Beispiel 2.6) erhaltene Lösung wurde bei RT in einem offenen Gefäß gelagert, um das Lösemittel abzudampfen. Nach 1 Woche wurde ein kristalliner Feststoff der Modifikation A erhalten.
5.5) Die nach Beispiel 2.7) erhaltene Lösung wurde bei RT in einem offenen Gefäß gelagert, um das Lösemittel abzudampfen. Nach 2 Tagen wurde ein kristalliner Feststoff der Modifikation B erhalten.
5.6) Die nach Beispiel 2.10) erhaltene Lösung wurde bei RT in einem offenen Gefäß gelagert, um das Lösemittel abzudampfen. Nach 2 Tagen wurde ein kristalliner Feststoff der Modifikation B erhalten.
5.7) Die nach Beispiel 4.1) erhaltene Lösung wurde bei RT in einem offenen Gefäß gelagert, um das Lösemittel abzudampfen. Nach 2 Tagen wurde ein kristalliner Feststoff der Modifikation A erhalten.
5.8) Die nach Beispiel 4.4) erhaltene Lösung wurde bei RT in einem offenen Gefäß gelagert, um das Lösemittel abzudampfen. Nach 6 Tagen wurde ein kristalliner Feststoff der Modifikation A erhalten.
5.9) Die nach Beispiel 4.5) erhaltene Lösung wurde bei RT in einem offenen Gefäß gelagert, um das Lösemittel abzudampfen. Nach 6 Tagen wurde ein kristalliner Feststoff der Modifikation B erhalten.
5.10) Die nach Beispiel 4.6) erhaltene Lösung wurde bei RT in einem offenen Gefäß gelagert, um das Lösemittel abzudampfen. Nach 6 Tagen wurde ein kristalliner Feststoff der Modifikation B erhalten.

### Beispiel 6:

Die kristalline Modifikation B von (1R,2R)-3-(3-Dimethylamino-1-ethyl-2-methylpropyl)-phenol zeigte beim Aufschlämmen in verschiedenen Lösemitteln keine Veränderung. Die Bildung von Solvaten mit den ausgewählten Lösemitteln konnte ausgeschlossen werden.
6.1) 200 mg ① wurden in 500 µl TBME suspendiert. Die Mischung wurde bei RT für die Dauer von 2 Tagen gerührt und der resultierende Feststoff wurde abfiltriert. Es wurde kristallines Pulver der Modifikation B erhalten.
6.2) 100 mg ① wurden in 500 µl Heptan/TBME (1:1) suspendiert. Die Mischung wurde bei RT für die Dauer von 2 Tagen gerührt und der resultierende Feststoff wurde abfiltriert. Es wurde kristallines Pulver der Modifikation B erhalten.
6.3) 100 mg ① wurden in 500 µl H₂O suspendiert. Die Mischung wurde bei RT für die Dauer von 2 Tagen gerührt und der resultierende Feststoff wurde abfiltriert. Es wurde kristallines Pulver der Modifikation B erhalten.
6.4) 100 mg ① wurden in 500 µl IPE suspendiert. Die Mischung wurde bei RT für die Dauer von 2 Tagen gerührt und der resultierende Feststoff wurde abfiltriert. Es wurde kristallines Pulver der Modifikation B erhalten.
6.5) 100 mg ① wurden in 500 µl H₂O/EtOH (1:1) suspendiert. Die Mischung wurde bei RT für die Dauer von 2 Tagen gerührt und der resultierende Feststoff wurde abfiltriert. Es wurde kristallines Pulver der Modifikation B erhalten.

### Beispiel 7:

Es wurde versucht, die amorphe Modifikation von (1R,2R)-3-(3-Dimethylamino-1-ethyl-2-methyl-propyl)-phenol durch Verdampfung, Lyophilisation oder Schmelzen herzustellen. Alle erhaltenen Proben der amorphen Modifikation kristallisieren innerhalb von Stunden.
7.1) 150 mg ① wurden in 3 ml MeOH gelöst. Das Lösemittel wurde am Rotationsverdampfer entfernt. Ein farbloses Öl blieb zurück. Der Rückstand wurde im Vakuum getrocknet. Es wurde Modifikation A erhalten.
7.2) 150 mg ① wurden in 2 ml 1,4-Dioxan gelöst. Das Lösemittel wurde mittels Gefriertrockner entfernt. Es wurde Modifikation A erhalten..
7.3) 150 mg ① wurden in 2 ml 1,4-Dioxan gelöst. Das Lösemittel wurde mittels Gefriertrockner (-85°C, 0,5 mbar) entfernt. Ein farbloser Rückstand blieb zurück, der spontan kristallisierte bevor es möglich war, eine PXRD-Analyse durchzuführen. Es wurde Modifikation B mit Spuren von Modifikation A erhalten.
7.4) 150 mg ① wurden bei 88-91 °C geschmolzen. Die Schmelze wurde mit Trockeneis gefroren. Es wurde ein amorpher Film erhalten, der innerhalb von 1 Stunde kristallisierte.

### Beispiel 8:

Der Einfluss mechanischer Beanspruchung durch Mahlen mit einer Kugelmühle (Typ Retsch MM200, Achatgefäß und Achatkugel mit 5 mm Durchmesser) sowie durch Druck bei der Herstellung einer Tablette wurde untersucht. Während der Druck bei der Tablettierung keinen Einfluss auf die Proben hat, wandelt sich Modifikation A beim Mahlen in Modifikation B um.
8.1) Auf einer IR Tablettenpresse (Druck 10 t, 30 min) wurde eine Tablette mit 100 mg ① hergestellt. Es wurde Modifikation B erhalten.
8.2) Auf einer IR Tablettenpresse (Druck 10 t, 30 min) wurde eine Tablette mit 100 mg Produkt der Modifikation A gemäß Beispiel 5.8 hergestellt. Es wurde Modifikation A erhalten.
8.3) 16 mg der kristallinen Modifikation B wurden in einer Kugelmühle (Schüttelfrequenz: 30 sec⁻¹, RT) wie folgt gemahlen: 2 mal 90 min, 1 mal 60 min, zwei mal 30 min Unterbrechung. Es wurde Modifikation B erhalten.
8.4) 15 mg der kristallinen Form A wurden in einer Kugelmühle (Schüttelfrequenz: 30 sec⁻¹, RT) wie folgt gemahlen: 2 mal 90 min, 1 mal 60 min, zwei mal 30 min Unterbrechung. Es wurde Modifikation B erhalten.

### Beispiel 9:

9.1) 20,5 mg Modifikation A und 20,9 mg Modifikation B wurden in 200 µl IPE suspendiert. Die Suspension wurde in einem Eppendorf Thermomixer bei RT über Nacht geschüttelt. Der erhaltene Feststoff wurde abfiltriert und mittels FT Raman charakterisiert. Es wurde Modifikation B erhalten.
9.2) 19,8 mg Modifikation A und 20,5 mg Modifikation B wurden in 300 µl Aceton/H₂O (8:2) suspendiert. Die Suspension wurde in einem Eppendorf Thermomixer bei RT über Nacht geschüttelt. Der erhaltene Feststoff wurde abfiltriert und mittels FT Raman charakterisiert. Es wurde Modifikation B erhalten.
9.3) 15 mg Modifikation A und 20,5 mg Modifikation B wurden in 1 ml Aceton/H₂O (8:2) suspendiert. Die Suspension wurde bei 5 °C für drei Tage gerührt. Der erhaltene Feststoff wurde abfiltriert und mittels FT Raman charakterisiert. Es wurde Modifikation B erhalten.
9.4) 20,5 mg Modifikation A und 20,9 mg Modifikation B wurden in 200 µl IPE suspendiert. Die Suspension wurde bei 50 °C über Nacht gerührt. Der erhaltene Feststoff wurde abfiltriert und mittels FT Raman charakterisiert. Es wurde Modifikation B erhalten.
9.5) 15 mg Modifikation A und 15 mg Modifikation B wurden in 1 ml Aceton/H₂O (8:2) suspendiert. Die Suspension wurde bei 50°C über Nacht gerührt. Der erhaltene Feststoff wurde abfiltriert und mittels FT Raman charakterisiert. Es wurde Modifikation B erhalten.
9.6) 20,5 mg Modifikation A und 20,9 mg Modifikation B wurden in 200 µl IPE suspendiert. Die Suspension wurde bei 50 °C über Nacht gerührt. Alle festen Bestandteile wurden gelöst. Nach dem Abkühlen auf RT fielen kleine Mengen eines farblosen Feststoffs aus. Das Lösemittel wurde unter Stickstoffstrom entfernt. Es wurde Modifikation B erhalten.

### Analytik - XRPD

Röntgenpulverdiffraktometrie (XRPD, x-ray powder diffraction):
XRPD Untersuchungen wurden mit einem STOE Stadi P
   Röntgenpulverdiffraktometer in Transmissionsgeometrie durchgeführt, wobei mittels Germanium Einkristall monochromatisierte CuKα₁-Strahlung verwendet wurde. D-Abstände werden von den 2θ-Werten berechnet, wobei die Wellenlänge von 1.54060 Å zu Grunde gelegt ist. Es gilt allgemein, dass die 2Θ Werte eine Fehlerrate von ± 0,2 ° in 2Θ besitzen. Der experimentelle Fehler bei den d-Abstandswerten ist daher abhängig vom Ort des Peaks.

### Modifikation A

Tabelle 1 zeigt die Peakliste der Modifikation A. Die Unsicherheit in den 2Θ Werten beträgt ± 0,2 ° in 2Θ, rel. I (oder auch RI) gibt die relative Intensität des jeweiligen Peaks an. Maximale Intensität ist 100.

**Tabelle 1:**

| 2Θ | rel. I | | 2Θ | rel. I | | 2Θ | rel. I | | 2Θ | rel. I | | 2Θ | rel. I |
|---|---|---|---|---|---|---|---|---|---|---|---|---|---|
| 13,71 | 30 | | 20,20 | 15 | | 24,75 | 9 | | 30,68 | 5 | | 36,24 | 2 |
| 14,11 | 9 | | 20,92 | 12 | | 25,33 | 18 | | 31,43 | 3 | | 36,54 | 1 |
| 14,80 | 45 | | 21,12 | 5 | | 27,32 | 5 | | 32,21 | 4 | | 36,87 | 1 |
| 15,58 | 100 | | 21,90 | 7 | | 27,55 | 6 | | 32,98 | 3 | | 37,06 | 2 |
| 16,31 | 3 | | 22,21 | 6 | | 28,05 | 2 | | 33,41 | 2 | | 37,48 | 2 |
| 16,89 | 18 | | 22,50 | 18 | | 28,37 | 3 | | 33,76 | 1 | | 37,87 | 1 |
| 17,79 | 37 | | 23,30 | 3 | | 29,62 | 1 | | 34,17 | 1 | | 38,64 | 3 |
| 18,45 | 34 | | 24,04 | 2 | | 29,90 | 5 | | 34,45 | 1 | | 39,48 | 2 |
| 19,07 | 8 | | 24,37 | 17 | | 30,28 | 1 | | 35,98 | 2 | | | |

Die Indizierung des Diffraktogramms der Form A mit dem Programm WinXPow Index (Version 2.03) der Firma STOE & Cie GmbH ergab die folgenden Gitterkonstanten, die in guter Übereinstimmung mit denen sind, die im Rahmen einer Einkristallstrukturbestimmung ermittelt wurden:
Orthorombisch, a = 12,92 Å, b = 11,98 Å, c= 8,98 Å, Zellvolumen 1391 Å³

### Modifikation B

Tabelle 2 zeigt die Peakliste der Modifikation B. Die Unsicherheit in den 2Θ Werten beträgt ± 0,2 ° in 2Θ, rel. I gibt die relative Intensität des jeweiligen Peaks an. Maximale Intensität ist 100.

**Tabelle 2:**

| 2Θ | rel. I | | 2Θ | rel. I | | 2Θ | rel. I | | 2Θ | rel. I | | 2Θ | rel. I |
|---|---|---|---|---|---|---|---|---|---|---|---|---|---|
| 14,11 | 47 | | 20,80 | 30 | | 25,36 | 8 | | 31,54 | 1 | | 37,12 | 2 |
| 14,44 | 35 | | 22,00 | 10 | | 27,58 | 9 | | 32,11 | 3 | | 37,32 | 2 |
| 16,08 | 100 | | 22,49 | 17 | | 27,79 | 5 | | 32,45 | 1 | | 37,75 | 1 |
| 17,17 | 42 | | 22,85 | 4 | | 29,06 | 19 | | 32,76 | 3 | | 38,13 | 1 |
| 17,43 | 33 | | 23,40 | 26 | | 29,89 | 10 | | 33,61 | 2 | | 38,72 | 2 |
| 18,67 | 5 | | 24,15 | 12 | | 30,11 | 5 | | 33,94 | 1 | | 39,63 | 3 |
| 18,81 | 37 | | 24,51 | 31 | | 30,31 | 2 | | 35,49 | 2 | | 40,01 | 1 |
| 19,50 | 1 | | 24,88 | 4 | | 31,00 | 6 | | 35,95 | 3 | | | |
| 20,24 | 15 | | 25,24 | 5 | | 31,17 | 4 | | 36,54 | 4 | | | |

Die Indizierung des Diffraktogramms der Form B mit dem Programm WinXPow Index (Version 2.03) der Firma STOE & Cie GmbH ergab die folgenden Gitterkonstanten, die in guter Übereinstimmung mit denen sind, die im Rahmen einer Einkristallstrukturbestimmung ermittelt wurden:
Orthorombisch, a = 12,54 Å, b = 12,27 Å, c= 9,10 Å, Zellvolumen 1400 Å³

### Modifikation C

Tabelle 3 zeigt die Peakliste der Modifikation C. Die Unsicherheit in den 2Θ Werten beträgt ± 0,2 ° in 2Θ, rel. I gibt die relative Intensität des jeweiligen Peaks an. Maximale Intensität ist 100.

**Tabelle 3:**

| 2Θ | rel. I | | 2Θ | rel. I | | 2Θ | rel. I | | 2Θ | rel. I | | 2Θ | rel. I | | 2Θ | rel. I |
|---|---|---|---|---|---|---|---|---|---|---|---|---|---|---|---|---|
| 8,10 | 4 | | 16,10 | 53 | | 20,23 | 32 | | 24,39 | 15 | | 28,48 | 4 | | 33,66 | 4 |
| 10,93 | 8 | | 16,43 | 100 | | 20,71 | 9 | | 24,92 | 39 | | 29,64 | 1 | | 35,52 | 3 |
| 11,83 | 4 | | 16,91 | 16 | | 20,94 | 12 | | 25,35 | 14 | | 29,94 | 7 | | 36,05 | 4 |
| 12,41 | 9 | | 17,32 | 5 | | 21,17 | 39 | | 26,22 | 17 | | 30,54 | 7 | | 36,64 | 3 |
| 13,71 | 14 | | 17,58 | 27 | | 21,90 | 6 | | 26,54 | 9 | | 30,68 | 5 | | 37,54 | 3 |
| 14,13 | 11 | | 17,82 | 27 | | 22,23 | 8 | | 26,72 | 10 | | 31,03 | 2 | | 38,45 | 2 |
| 14,82 | 24 | | 18,01 | 30 | | 22,52 | 11 | | 27,33 | 4 | | 31,52 | 3 | | 39,15 | 3 |
| 15,34 | 38 | | 18,46 | 25 | | 23,32 | 2 | | 27,63 | 5 | | 32,29 | 3 | | 40,05 | 6 |
| 15,59 | 58 | | 19,05 | 33 | | 24,12 | 4 | | 27,84 | 7 | | 32,93 | 5 | | | |

### Analytik - DSC

Differential Scanning Calorimetrie (DSC): Gerätebezeichnung Perkin Elmer DSC 7 oder Perkin Elmer Pyris 1. Sofern nicht anders angegeben wurden die Proben in einem versiegelten Goldtiegel eingewogen. Die Messung erfolgte mit Stickstoffstrom in einem Temperaturbereich von -50 °C bis 250 °C mit einer Heizrate von 10 °C/min. Bei den im Zusammenhang mit DSC Untersuchungen angegebenen Temperaturen handelt es sich, sofern nicht anders angegeben, um die Temperaturen der Peakmaxima (Peaktemperatur T_{P}). Mit T_{O} werden Onsettemperaturen von Peaks bezeichnet.

### DSC

- Modifikation A: T_{O} 77,83 °C; T_{P} 79,46 °C; J/g 107,03
- Modifikation B: T_{O} 88,60 °C; T_{P} 89,76 °C; J/g 114,67
- Modifikation C: T_{O} 78,72 °C; T_{P} 81,00 °C; J/g 110,74
T_{O} 88,36 °C; T_{P} 89,17 °C; J/g 0,57

### Analytik - FT-Raman-Spektroskopie

Die kristallinen Modifikationen A und B von (1R,2R)-3-(3-Dimethylamino-1-ethyl-2-methyl-propyl)-phenol wurden jeweils mit Hilfe von Fourier-Transform-(FT)-RAMAN-Spektrometrie charakterisiert.

Hierzu wurden auf einem Bruker RFS100 RAMAN-Spektrometer (Nd-YAG 100 mW Laser, Anregung 1064 nm, Ge-Detektor, 64 Scans, 25-3500 cm⁻¹, Auflösung 2 cm⁻¹) die FT-Raman-Spektren aufgenommen.

### Analytik - TG-FTIR

Die kristallinen Modifikationen A und B von (1R,2R)-3-(3-Dimethylamino-1-ethyl-2-methyl-propyl)-phenol wurden jeweils mit Hilfe von Thermogravimetrischer Fourier-Transform-Infrarot-Spektroskopie (TG-FTIR) charakterisiert.

Hierzu wurden die entsprechenden Spektren mit einer Netzsch Thermo-Microwaage TG 209 sowie einem Bruker FT-IR Spektrometer Vector 22 aufgenommen (Aluminiumtiegel (offen oder mit Mikroöffnung), Stickstoffatmosphäre, Aufheizrate 10 °C/min, 25-250°C)

Die TG-FTIR-Untersuchungen ergaben, dass sich beide Modifikationen oberhalb von 160 °C zersetzen.

### Analytik - DVS

Die kristallinen Modifikationen A und B von (1R,2R)-3-(3-Dimethylamino-1-ethyl-2-methyl-propyl)-phenol wurden jeweils mittels dynamischer Wasserdampfsorption (DVS, Dynamic vapor sorption) charakterisiert. Die Untersuchungen wurden im dynamischen Modus (5% relative Luftfeuchtigkeit/Stunde) aufgenommen.

Die DVS Zyklen sind reversibel. Bei einer Temperatur von 25 °C wurden Massenänderungen von 0,8 % für Modifikation A und 0,3 % für Modifikation B gefunden. Beide Modifikationen sind nicht bis höchstens leicht hygroskopisch.

### Analytik - Auflösungsgeschwindikgeit

Zur Untersuchung der Auflösungsgeschwindigkeit der Modifikationen A und B in Wasser wurden zwei verschiedene Bestimmungen durchgeführt.

In der ersten Bestimmung wurde jeweils eine Suspension der Modifikation A bzw. B in Wasser gerührt ohne Berücksichtigung der Teilchengrößenverteilung. Unter diesen Bedingungen beeinflusst die Teilchengröße das Ergebnis. Obwohl Form B die bei RT stabilere Form darstellt, löst sie sich schneller auf.

In der zweiten Bestimmung wurde eine frische Probe der Modifikation A hergestellt und für beide Modifikationen A und B Tabletten hergestellt. Die jeweilige Form wurde durch die Tablettierung nicht beeinflusst, dennoch zeigten beiden Proben eine Auflösungsgeschwindigkeit von 0,003 mg/min cm². Untersuchung der Proben mit FT-Raman ergab, dass Form A während der Bestimmung in Form B umgewandelt wurde.

## Patentansprüche

1. Kristalline Modifikation A von (1 R,2R)-3-(3-Dimethylamino-1-ethyl-2-methyl-propyl)-phenol umfassend einen Röntgenbeugungsreflex bei 15,58±0,20 (2Θ), zusätzlich mindestens einen Röntgenbeugungsreflex ausgewählt aus der Gruppe bestehend aus 28,37±0,20 (2Θ) und 34,45±0,20 (2Θ) und zusätzlich mindestens einen Röntgenbeugungsreflex ausgewählt aus der Gruppe bestehend aus 13,71±0,20 (2Θ), 14,80±0,20 (2Θ), 16,89±0,20 (2Θ), 17,79±0,20 (2Θ), 18,45±0,20 (2Θ), 20,20±0,20 (2Θ), 20,92±0,20 (2Θ), 22,50±0,20 (2Θ), 24,37±0,20 (2Θ) und 25,33±0,20 (2Θ).

2. Kristalline Modifikation A gemäß Anspruch 1, **dadurch gekennzeichnet, dass** sie zusätzlich mindestens einen Röntgenbeugungsreflex umfasst ausgewählt aus der Gruppe bestehend aus 14,11±0,20 (2Θ), 19,07±0,20 (2Θ), 21,12±0,20 (2Θ), 21,90±0,20 (2Θ), 22,21±0,20 (2Θ), 24,75±0,20 (2Θ), 27,32±0,20 (2Θ), 27,55±0,20 (2Θ), 29,90±0,20 (2Θ) und 30,68±0,20 (2Θ).

3. Kristalline Modifikation A gemäß einem der Ansprüche 1 oder 2, **dadurch gekennzeichnet, dass** sie mindestens einen Röntgenbeugungsreflex ausgewählt aus der Gruppe bestehend aus 8,10±0,20 (2Θ), 10,93±0,20 (2Θ), 11,83±0,20 (2Θ), 12,41±0,20 (2Θ), 26,22±0,20 (2Θ), 26,54±0,20 (2Θ) und 26,72±0,20 (2Θ) nicht umfasst.

4. Kristalline Modifikation A gemäß einem der Ansprüche 1-3, **dadurch gekennzeichnet, dass** sie in der DSC eine Endothermie im Bereich von 75 bis 84 °C aufweist.

5. Verfahren zur Herstellung der kristallinen Modifikation A gemäß einem der Ansprüche 1-4 umfassend die Schritte
(a) Einengen einer Lösung von (1R,2R)-3-(3-Dimethylamino-1-ethyl-2-methyl-propyl)-phenol, wobei das Lösemittel Methanol, Ethanol, 1-Propanol, 2-Propanol, Essigester, Aceton, Ethylmethylketon, Diethylether, tert-Butylmethylether, 1,4-Dioxan, Tetrahydrofuran, Acetonitril, Dichlormethan, Toluol, Dimethylformamid oder Dimethylsulfoxid ist, und
(b) Lagern des gemäß Schritt (a) erhaltenen Rückstands bei einer Temperatur von > 5 °C.

6. Kristalline Modifikation B von (1R,2R)-3-(3-Dimethylamino-1-ethyl-2-methylpropyl)-phenol umfassend einen Röntgenbeugungsreflex bei 29,06±0,20 (2Θ), zusätzlich mindestens einen Röntgenbeugungsreflex ausgewählt aus der Gruppe bestehend aus 19,50±0,20 (2Θ), 35,49±0,20 (2Θ) und 40,01±0,20 (2Θ) und zusätzlich mindestens einen Röntgenbeugungsreflex ausgewählt aus der Gruppe bestehend aus 14,11±0,20 (2Θ), 14,44±0,20 (2Θ), 16,08±0,20 (2Θ), 17,17±0,20 (2Θ), 17,43±0,20 (2Θ), 18,81 ±0,20 (2Θ), 20,24±0,20 (2Θ), 20,80±0,20 (2Θ), 22,00 ±0,20 (2Θ), 22,49±0,20 (2Θ), 23,40±0,20 (2Θ), 24,15±0,20 (2Θ), 24,51±0,20 (2Θ) und 29,89±0,20 (2Θ).

7. Kristalline Modifikation B gemäß Anspruch 6, **dadurch gekennzeichnet, dass** sie zusätzlich mindestens einen Röntgenbeugungsreflex umfasst ausgewählt aus der Gruppe bestehend aus 18,67±0,20 (2Θ), 25,24±0,20 (2Θ), 25,36±0,20 (2Θ), 27,58±0,20 (2Θ), 27,79±0,20 (2Θ), 30,11±0,20 (2Θ) und 31,00±0,20 (2Θ).

8. Kristalline Modifikation B gemäß einem der Ansprüche 6 oder 7, **dadurch gekennzeichnet, dass** sie mindestens einen Röntgenbeugungsreflex ausgewählt aus der Gruppe bestehend aus 8,10±0,20 (2Θ), 10,93±0,20 (2Θ), 11,83±0,20 (2Θ), 12,41±0,20 (2Θ), 26,22±0,20 (2Θ), 26,54±0,20 (2Θ) und 26,72±0,20 (2Θ) nicht umfasst.

9. Kristalline Modifikation B gemäß einem der Ansprüche 6-8, **dadurch gekennzeichnet, dass** sie in der DSC eine Endothermie im Bereich von 87-93°C aufweist.

10. Verfahren zur Herstellung der kristallinen Modifikation B gemäß einem der Ansprüche 6-8 umfassend die Schritte
(a) Einengen einer Lösung von (1R,2R)-3-(3-Dimethylamino-1-ethyl-2-methyl-propyl)-phenol, wobei das Lösemittel Methanol, Ethanol, 1-Propanol, 2-Propanol, Essigester, Aceton, Ethylmethylketon, Diethylether, tert-Butylmethylether, 1,4-Dioxan, Tetrahydrofuran, Acetonitril, Dichlormethan, Toluol, Dimethylformamid oder Dimethylsulfoxid ist, und
(b1) Lagern des gemäß Schritt (a) erhaltenen Rückstands bei einer Temperatur von ≤ 5 °C, oder
(b2) Suspendieren des gemäß Schritt (a) erhaltenen Rückstands und Rühren dieser Suspension.

11. Verfahren zur Herstellung der kristallinen Modifikation B gemäß einem der Ansprüche 6-8 umfassend den Schritt
(a) Präzipitieren von (1R,2R)-3-(3-Dimethylamino-1-ethyl-2-methyl-propyl)-phenol aus Lösung, wobei das Lösemittel Methanol, Ethanol, 1-Propanol, 2-Propanol, Essigester, Aceton, Ethylmethylketon, Diethylether, tert-Butylmethylether, 1,4-Dioxan, Tetrahydrofuran, Acetonitril, Dichlormethan, Toluol, Dimethylformamid oder Dimethylsulfoxid ist.

12. Kristalline Modifikation C von (1R,2R)-3-(3-Dimethylamino-1-ethyl-2-methylpropyl)-phenol umfassend mindestens einen Röntgenbeugungsreflex ausgewählt aus der Gruppe bestehend aus 10,93±0,20 (2Θ), 12,41±0,20 (2Θ) und 26,22±0,20 (2Θ), zusätzlich mindestens einen Röntgenbeugungsreflex ausgewählt aus der Gruppe bestehend aus 8,10±0,20 (2Θ), 11,83±0,20 (2Θ), 26,54±0,20 (2Θ) und 26,72±0,20 (2Θ) und zusätzlich mindestens einen Röntgenbeugungsreflex ausgewählt aus der Gruppe bestehend aus 13,71±0,20 (2Θ), 14,13±0,20 (2Θ), 14,82±0,20 (2Θ), 15,34±0,20 (2Θ), 15,59±0,20 (2Θ), 16,10±0,20 (2Θ), 16,43±0,20 (2Θ), 16,91±0,20 (2Θ), 17,32±0,20 (2Θ), 17,58±0,20 (2Θ), 17,82±0,20 (2Θ), 18,01±0,20 (2Θ), 18,46±0,20 (2Θ), 19,05±0,20 (2Θ), 20,23±0,20 (2Θ), 20,71±0,20 (2Θ), 20,94±0,20 (2Θ), 21,17±0,20 (2Θ), 21,90±0,20 (2Θ), 22,23±0,20 (2Θ), 22,52±0,20 (2Θ), 23,32±0,20 (2Θ), 24,12±0,20 (2Θ), 24,39±0,20 (2Θ), 24,92±0,20 (2Θ), 25,35±0,20 (2Θ), 27,33±0,20 (2Θ), 27,63±0,20 (2Θ), 27,84±0,20 (2Θ), 28,48±0,20 (2Θ), 29,64±0,20 (2Θ), 29,94±0,20 (2Θ), 30,54±0,20 (2Θ), 30,68±0,20 (2Θ), 31,03±0,20 (2Θ), 31,52±0,20 (2Θ), 32,29±0,20 (2Θ), 32,93±0,20 (2Θ), 33,66±0,20 (2Θ), 35,52±0,20 (2Θ), 36,05±0,20 (2Θ), 36,64±0,20 (2Θ), 37,54±0,20 (2Θ), 38,45±0,20 (2Θ), 39,15±0,20 (2Θ) und 40,05±0,20 (2Θ).

13. Kristalline Modifikation C gemäß Anspruch 12, **dadurch gekennzeichnet, dass** sie bei DSC Untersuchungen eine Endothermie mit Peaktemperatur bei 75-84°C aufweist und/oder eine Endothermie mit Peaktemperatur bei 87-93°C.

14. Verfahren zur Herstellung der kristallinen Modifikation C gemäß einem der Ansprüche 12 oder 13 umfassend die Schritte
(a) Schütteln einer Suspension enthaltend die kristalline Modifikation A und/oder die kristalline Modifikation B von (1R,2R)-3-(3-Dimethylamino-1-ethyl-2-methyl-propyl)-phenol, wobei das Suspensionsmedium Methanol oder Toluol ist,
und
(b) Verdampfen des Suspensionsmediums im Luftstrom.

15. Pharmazeutische Zusammensetzung umfassend eine kristalline Modifikation von (1R,2R)-3-(3-Dimethylamino-1-ethyl-2-methyl-propyl)-phenol gemäß einem oder mehreren der Ansprüche 1-4, 6-9 und 12-13 sowie einen pharmazeutisch verträglichen Träger.

16. Pharmazeutische Darreichungsform umfassend eine pharmazeutische Zusammensetzung nach Anspruch 15.

17. Kristalline Modifikation von (1R,2R)-3-(3-Dimethylamino-1-ethyl-2-methyl-propyl)-phenol gemäß einem oder mehreren der Ansprüche 1-4, 6-9 und 12-13 als Arzneimittel.

18. Kristalline Modifikation von (1R,2R)-3-(3-Dimethylamino-1-ethyl-2-methylpropyl)-phenol gemäß einem oder mehreren der Ansprüche 1-4, 6-9 und 12-13 zur Verwendung in der Bekämpfung von Schmerz.

## Claims

1. Crystalline modification A of (1R,2R)-3-(3-dimethylamino-1-ethyl-2-methylpropyl)phenol comprising an X-ray diffraction reflection at 15.58±0.20 (2Θ), in addition at least one X-ray diffraction reflection selected from the group consisting of 28.37±0.20 (2Θ) and 34.45±0.20 (2Θ) and in addition at least one X-ray diffraction reflection selected from the group consisting of 13.71±0.20 (2Θ), 14.80±0.20 (2Θ), 16.89±0.20 (2Θ), 17.79±0.20 (2Θ), 18.45±0.20 (2Θ), 20.20±0.20 (2Θ), 20.92±0.20 (2Θ), 22.50±0.20 (2Θ), 24.37±0.20 (2Θ) and 25.33±0.20 (2Θ).

2. Crystalline modification A according to Claim 1, **characterized in that** it comprises in addition at least one X-ray diffraction reflection selected from the group consisting of 14.11+0.20 (2Θ), 19.07±0.20 (2Θ), 21.12±0.20 (2Θ), 21.90±0.20 (2Θ), 22.21±0.20 (2Θ), 24.75+0.20 (2Θ), 27.32±0.20 (2Θ), 27.55±0.20 (2Θ), 29.90±0.20 (2Θ) and 30.68±0.20 (2Θ).

3. Crystyalline modification A according to either of Claims 1 and 2, **characterized in that** it does not comprise at least one X-ray diffraction reflection selected from the group consisting of 8.10±0.20 (2Θ), 10.93+0.20 (2Θ) 11.83+0.20 (2Θ), 12.41±0.20 (2Θ), 26.22±0.20 (2Θ), 26.54±0.20 (2Θ) and 26.72±0.20 (2Θ).

4. Crystalline modification A according to any of Claims 1-3, **characterized in that** it has an endotherm in the range of 75 to 84°C in the DSC.

5. Method for preparing crystalline modification A according to any of Claims 1-4 comprising the steps of
(a) concentrating a solution of (1R,2R)-3-(3-dimethylamino-1-ethyl-2-methylpropyl)phenol, wherein the solvent is methanol, ethanol, 1-propanol, 2-propanol, ethyl acetate, acetone, ethyl methyl ketone, diethyl ether, tert-butyl methyl ether, 1,4-dioxane, tetrahydrofuran, acetonitrile, dichloromethane, toluene, dimethylformamide or dimethyl sulphoxide, and
(b) storing the residue obtained according to step (a) at a temperature of >5°C.

6. Crystalline modification B of (1R,2R)-3-(3-dimethylamino-1-ethyl-2-methylpropyl)phenol comprising an X-ray diffraction reflection at 29.06+0.20 (2Θ), in addition at least one X-ray diffraction reflection selected from the group consisting of 19.50±0.20 (2Θ), 35.49±0.20 (2Θ) and 40.01±0.20 (2Θ) and in addition at least one X-ray diffraction reflection selected from the group consisting of 14.11±0.20 (2Θ), 14.44±0.20 (2Θ), 16.08±0.20 (2Θ), 17.17±0.20 (2Θ), 17.43±0.20 (2Θ), 18.81±0.20 (2Θ), 20.24±0.20 (2Θ), 20.80±0.20 (2Θ), 22.00±0.20 (2Θ), 22.49±0.20 (2Θ), 23.40±0.20 (2Θ), 24.15±0.20 (2Θ), 24.51±0.20 (2Θ) and 29.89±0.20 (2Θ).

7. Crystalline modification B according to Claim 6, **characterized in that** it comprises in addition at least one X-ray diffraction reflection selected from the group consisting of 18.67+0.20 (2Θ), 25.24±0.20 (2Θ), 25.36±0.20 (2Θ), 27.58+0.20 (2Θ), 27.79±0.20 (2Θ), 30.11±0.20 (2Θ) and 31.00±0.20 (2Θ).

8. Crystalline modification B according to either of Claims 6 and 7, **characterized in that** it does not comprise at least one X-ray diffraction reflection selected from the group consisting of 8.10+0.20 (2Θ), 10.93±0.20 (2Θ), 11.83±0.20 (2Θ), 12.41+0.20 (2Θ), 26.22±0.20 (2Θ), 26.54±0.20 (2Θ) and 26.72±0.20 (2Θ).

9. Crystalline modification B according to any of Claims 6-8, **characterized in that** it has an endotherm in the range of 87 to 93°C in the DSC.

10. Method for preparing crystalline modification B according to any of Claims 6-8 comprising the steps of
(a) concentrating a solution of (1R,2R)-3-(3-dimethylamino-1-ethyl-2-methylpropyl)phenol, wherein the solvent is methanol, ethanol, 1-propanol, 2-propanol, ethyl acetate, acetone, ethyl methyl ketone, diethyl ether, tert-butyl methyl ether, 1,4-dioxane, tetrahydrofuran, acetonitrile, dichloromethane, toluene, dimethylformamide or dimethyl sulphoxide, and
(b1) storing the residue obtained according to step (a) at a temperature of ≤5°C, or
(b2) suspending the residue obtained according to step (a) and stirring this suspension.

11. Method for preparing crystalline modification B according to any of Claims 6-8 comprising the step of
(a) precipitating (1R,2R)-3-(3-dimethylamino-1-ethyl-2-methylpropyl)phenol from solution, wherein the solvent is methanol, ethanol, 1-propanol, 2-propanol, ethyl acetate, acetone, ethyl methyl ketone, diethyl ether, tert-butyl methyl ether, 1,4-dioxane, tetrahydrofuran, acetonitrile, dichloromethane, toluene, dimethylformamide or dimethyl sulphoxide.

12. Crystalline modification C of (1R,2R)-3-(3-dimethylamino-1-ethyl-2-methylpropyl)phenol comprising at least one X-ray diffraction reflection selected from the group consisting of 10.93±0.20 (2Θ), 12.41±0.20 (2Θ) and 26.22±0.20 (2Θ), in addition at least one X-ray diffraction reflection selected from the group consisting of 8.10±0.20 (2Θ), 11.83±0.20 (2Θ), 26.54±0.20 (2Θ) and 26.72±0.20 (2Θ) and in addition at least one X-ray diffraction reflection selected from the group consisting of 13.71±0.20 (2Θ), 14.13+0.20 (2Θ), 14.82±0.20 (2Θ), 15.34±0.20 (2Θ), 15.59±0.20 (2Θ), 16.10±0.20 (2Θ), 16.43±0.20 (2Θ), 16.91±0.20 (2Θ), 17.32±0.20 (2Θ), 17.58±0.20 (2Θ), 17.82±0.20 (2Θ), 18.01±0.20 (2Θ), 18.46±0.20 (2Θ), 19.05±0.20 (2Θ), 20.23+0.20 (2Θ), 20.71+0.20 (2Θ), 20.94+0.20 (2Θ), 21.17+0.20 (2Θ), 21.90±0.20 (2Θ), 22.23±0.20 (2Θ), 22.52±0.20 (2Θ), 23.32±0.20 (2Θ), 24.12±0.20 (2Θ), 24.39±0.20 (2Θ), 24.92±0.20 (2Θ), 25.35±0.20 (2Θ), 27.33±0.20 (2Θ), 27.63±0.20 (2Θ), 27.84±0.20 (2Θ), 28.48±0.20 (2Θ), 29.64±0.20 (2Θ), 29.94±0.20 (2Θ), 30.54±0.20 (2Θ), 30.68±0.20 (2Θ), 31.03±0.20 (2Θ), 31.52±0.20 (2Θ), 32.29±0.20 (2Θ), 32.93±0.20 (2Θ), 33.66±0.20 (2Θ), 35.52±0.20 (2Θ), 36.05±0.20 (2Θ), 36.64±0.20 (2Θ), 37.54±0.20 (2Θ), 38.45±0.20 (2Θ), 39.15±0.20 (2Θ) and 40.05±0.20 (2Θ).

13. Crystalline modification C according to Claim 12, **characterized in that** it has an endotherm with peak temperature at 75-84°C and/or an endotherm with peak temperature at 87-93°C in DSC investigations.

14. Method for preparing crystalline modification C according to either of Claims 12 and 13 comprising the steps of
(a) shaking a suspension containing crystalline modification A and/or crystalline modification B of (1R,2R)-3-(3-dimethylamino-1-ethyl-2-methylpropyl)phenol, wherein the suspension medium is methanol or toluene,
and
(b) evaporating the suspension medium in a stream of air.

15. Pharmaceutical composition comprising a crystalline modification of (1R,2R)-3-(3-dimethylamino-1-ethyl-2-methylpropyl)phenol according to one or more of Claims 1-4, 6-9 and 12-13 and a pharmaceutically compatible carrier.

16. Pharmaceutical dosage form comprising a pharmaceutical composition according to Claim 15.

17. Crystalline modification of (1R,2R)-3-(3-dimethylamino-1-ethyl-2-methylpropyl)phenol according to one or more of Claims 1-4, 6-9 and 12-13 as a medicament.

18. Crystalline modification of (1R,2R)-3-(3-dimethylamino-1-ethyl-2-methylpropyl)phenol according to one or more of Claims 1-4, 6-9 and 12-13 for use in combatting pain.

## Revendications

1. Modification cristalline A de (1R,2R)-3-(3-diméthylamino-1-éthyl-2-méthyl-propyl)-phénol comprenant une réflexion de diffraction de rayons X à 15,58±0,20 (2θ), en outre au moins une réflexion de diffraction de rayons X choisie dans le groupe formé par 28,37±0,20 (2θ) et 34,45±0,20 (2θ) et en outre au moins une réflexion de diffraction de rayons X choisie dans le groupe formé par 13,71±0,20 (2θ), 14,80±0,20 (2θ), 16,89±0,20 (2θ), 17,79±0,20 (2θ), 18,45±0,20 (2θ), 20,20±0,20 (2θ), 20,92±0,20 (2θ), 22,50±0,20 (2θ), 24,37±0,20 (2θ) et 25,33±0,20 (2θ).

2. Modification cristalline A selon la revendication 1, **caractérisée en ce qu'**elle comprend en outre au moins une réflexion de diffraction de rayons X choisie dans le groupe formé par 14,11±0,20 (2θ), 19,07±0,20 (2θ), 21,12±0,20 (2θ), 21,90±0,20 (2θ), 22,21±0,20 (2θ), 24,75±0,20 (2θ), 27,32±0,20 (2θ), 27,55±0,20 (2θ), 29,90±0,20 (2θ) et 30,68±0,20 (2θ).

3. Modification cristalline A selon l'une quelconque des revendications 1 ou 2, **caractérisée en ce qu'**elle ne comprend pas au moins une réflexion de diffraction de rayons X choisie dans le groupe formé par 8,10±0,20 (2θ), 10,93±0,20 (2θ), 11,83±0,20 (2θ), 12,41±0,20 (2θ), 26,22±0,20 (2θ), 26,54±0,20 (2θ) et 26,72±0,20 (2θ).

4. Modification cristalline A selon l'une quelconque des revendications 1-3, **caractérisée en ce qu'**elle présente une endothermie en DSC (calorimétrie différentielle par balayage) dans la plage de 75 à 84°C.

5. Procédé pour la préparation de la modification cristalline A selon l'une quelconque des revendications 1-4, comprenant les étapes consistant à
(a) concentrer une solution de (1R,2R)-3-(3-diméthylamino-1-éthyl-2-méthyl-propyl)-phénol, le solvant étant le méthanol, l'éthanol, le 1-propanol, le 2-propanol, l'ester acétique, l'acétone, l'éthylméthylcétone, le diéthyléther, le tert-butylméthyléther, le 1,4-dioxane, le tétrahydrofuranne, l'acétonitrile, le dichlorométhane, le toluène, le diméthylformamide ou le diméthylsulfoxyde et
(b) entreposer le résidu obtenu selon l'étape (a) à une température > 5°C.

6. Modification cristalline B de (1R,2R)-3-(3-diméthylamino-1-éthyl-2-méthyl-propyl)-phénol comprenant une réflexion de diffraction de rayons X à 29,06±0,20 (2θ), en outre au moins une réflexion de diffraction de rayons X choisie dans le groupe formé par 19,50±0,20 (2θ), 35,49±0,20 (2θ) et 40,01±0,20 (2θ) et en outre au moins une réflexion de diffraction de rayons X choisie dans le groupe formé par 14,11±0,20 (2θ), 14,44±0,20 (2θ), 16,08±0,20 (2θ), 17,17±0,20 (2θ), 17,43±0,20 (2θ), 18,81±0,20 (2θ), 20,24±0,20 (2θ), 20,80±0,20 (2θ), 22,00±0,20 (2θ), 22,49±0,20 (2θ), 23,40±0,20 (2θ), 24,15±0,20 (2θ), 24,51±0,20 (2θ) et 29,89±0,20 (2θ).

7. Modification cristalline B selon la revendication 6, **caractérisée en ce qu'**elle comprend en outre au moins une réflexion de diffraction de rayons X choisie dans le groupe formé par 18,67±0,20 (2θ), 25,24±0,20 (2θ), 25,36±0,20 (2θ), 27,58±0,20 (2θ), 27,79±0,20 (2θ), 30,11±0,20 (2θ) et 31,00±0,20 (2θ).

8. Modification cristalline B selon l'une quelconque des revendications 6 ou 7, **caractérisée en ce qu'**elle ne comprend pas au moins une réflexion de diffraction de rayons X choisie dans le groupe formé par 8,10±0,20 (2θ), 10,93±0,20 (2θ), 11,83±0,20 (2θ), 12,41±0,20 (2θ), 26,22±0,20 (2θ), 26,54±0,20 (2θ) et 26,72±0,20 (2θ) .

9. Modification cristalline B selon l'une quelconque des revendications 6-8, **caractérisée en ce qu'**elle présente une endothermie en DSC dans la plage de 87-93°C.

10. Procédé pour la préparation de la modification cristalline B selon l'une quelconque des revendications 6-8, comprenant les étapes consistant à
(a) concentrer une solution de (1R,2R)-3-(3-diméthylamino-1-éthyl-2-méthyl-propyl)-phénol, le solvant étant le méthanol, l'éthanol, le 1-propanol, le 2-propanol, l'ester acétique, l'acétone, l'éthylméthylcétone, le diéthyléther, le tert-butylméthyléther, le 1,4-dioxane, le tétrahydrofuranne, l'acétonitrile, le dichlorométhane, le toluène, le diméthylformamide ou le diméthylsulfoxyde et
(b1) entreposer le résidu obtenu selon l'étape (a) à une température ≤ 5°C ou
(b2) mettre en suspension le résidu obtenu selon l'étape (a) et agiter cette suspension.

11. Procédé pour la préparation de la modification cristalline B selon l'une quelconque des revendications 6-8, comprenant l'étape consistant à
(a) précipiter le (1R,2R)-3-(3-diméthylamino-1-éthyl-2-méthyl-propyl)-phénol à partir d'une solution, le solvant étant le méthanol, l'éthanol, le 1-propanol, le 2-propanol, l'ester acétique, l'acétone, l'éthylméthylcétone, le diéthyléther, le tert-butylméthyléther, le 1,4-dioxane, le tétrahydrofuranne, l'acétonitrile, le dichlorométhane, le toluène, le diméthylformamide ou le diméthylsulfoxyde.

12. Modification cristalline C de (1R,2R)-3-(3-diméthylamino-1-éthyl-2-méthyl-propyl)-phénol comprenant au moins une réflexion de diffraction de rayons X choisie dans le groupe formé par 10,93±0,20 (2θ), 12,41±0,20 (2θ) et 26,22±0,20 (2θ), en outre au moins une réflexion de diffraction de rayons X choisie dans le groupe formé par 8,10±0,20 (2θ), 11,83±0,20 (2θ), 26,54±0,20 (2θ) et 26,72±0,20 (2θ) et en outre au moins une réflexion de diffraction de rayons X choisie dans le groupe formé par 13,71±0,20 (2θ), 14,13±0,20 (2θ), 14,82±0,20 (2θ), 15,34±0,20 (2θ), 15,59±0,20 (2θ), 16,10±0,20 (2θ), 16,43±0,20 (2θ), 16,91±0,20 (2θ), 17,32±0,20 (2θ), 17,58±0,20 (2θ), 17,82±0,20 (2θ), 18,01±0,20 (2θ), 18,46±0,20 (2θ), 19,05±0,20 (2θ), 20,23±0,20 (2θ), 20,71±0,20 (2θ), 20,94±0,20 (2θ), 21,17±0,20 (2θ), 21,90±0,20 (2θ), 22,23±0,20 (2θ), 22,52±0,20 (2θ), 23,32±0,20 (2θ), 24,12±0,20 (2θ), 24,39±0,20 (2θ), 24,92±0,20 (2θ), 25,35±0,20 (2θ), 27,33±0,20 (2θ), 27,63±0,20 (2θ), 27,84±0,20 (2θ), 28,48±0,20 (2θ), 29,64±0,20 (2θ), 29,94±0,20 (2θ), 30,54±0,20 (2θ), 30,68±0,20 (2θ), 31,03±0,20 (2θ), 31,52±0,20 (2θ), 32,29±0,20 (2θ), 32,93±0,20 (2θ), 33,66±0,20 (2θ), 35,52±0,20 (2θ), 36,05±0,20 (2θ), 36,64±0,20 (2θ), 37,54±0,20 (2θ), 38,45±0,20 (2θ), 39,15±0,20 (2θ) et 40,05±0,20 (2θ).

13. Modification cristalline C selon la revendication 12, **caractérisée en ce qu'**elle présente, lors d'analyses par DSC, une endothermique à une température de pic à 75-84°C et/ou une endothermie à une température de pic à 87-93°C.

14. Procédé pour la préparation de la modification cristalline C selon l'une quelconque des revendications 12 ou 13, comprenant les étapes consistant à
(a) agiter une suspension contenant la modification cristalline A et/ou la modification cristalline B de (1R,2R)-3-(3-diméthylamino-1-éthyl-2-méthyl-propyl)-phénol, le milieu de suspension étant le méthanol ou le toluène, et
(b) évaporer le milieu de suspension dans un flux d'air.

15. Composition pharmaceutique comprenant une modification cristalline de (1R,2R)-3-(3-diméthylamino-1-éthyl-2-méthyl-propyl)-phénol selon l'une ou plusieurs des revendications 1-4, 6-9 et 12-13 ainsi qu'un support pharmaceutiquement acceptable.

16. Forme galénique pharmaceutique comprenant une composition pharmaceutique selon la revendication 15.

17. Modification cristalline de (1R,2R)-3-(3-diméthylamino-1-éthyl-2-méthyl-propyl)-phénol selon l'une ou plusieurs des revendications 1-4, 6-9 et 12-13 comme médicament.

18. Modification cristalline de (1R,2R)-3-(3-diméthylamino-1-éthyl-2-méthyl-propyl)-phénol selon l'une ou plusieurs des revendications 1-4, 6-9 et 12-13 pour une utilisation dans la lutte contre la douleur.
